(19) Europäisches Patentamt
European Patent Office
Office européen des brevets

(11) Publication number: **0 174 835**
**A1**

(12) **EUROPEAN PATENT APPLICATION**

(21) Application number: 85306432.7

(22) Date of filing: 10.09.85

(51) Int. Cl.⁴: C 12 N 9/48
C 12 N 9/60, C 12 N 15/00
C 12 N 1/16, C 12 N 5/00
//C12R1:865

(30) Priority: 11.09.84 US 648663
19.10.84 US 663025

(43) Date of publication of application:
19.03.86 Bulletin 86/12

(84) Designated Contracting States:
BE CH DE FR GB IT LI NL SE

(71) Applicant: THE UPJOHN COMPANY
301 Henrietta Street
Kalamazoo, Michigan 49001(US)

(72) Inventor: Gill, Gurnam S.
c/o The Upjohn Company 301 Henrietta Street
Kalamazoo Michigan 49001(US)

(74) Representative: Perry, Robert Edward et al,
GILL JENNINGS & EVERY 53-64 Chancery Lane
London WC2A 1HN(GB)

(54) **Human tissue plasminogen activator and recombinant DNA compounds.**

(57) A human tissue plasminogen activator, and analogues thereof, which are the product of expression of recombinant DNA in yeast. The products are useful as fibrinolytic agents.

EP 0 174 835 A1

This invention relates to a human tissue plasminogen activator (t-PA) and analogues thereof, and to recombinant DNA (rDNA) compounds. The invention relates also to yeasts having the recombinant compounds therein. The t-PA and its analogues can have fibrinolytic activity, and are thus suitable for the treatment of diseases having an underlying etiology known to benefit from this activity.

Wilson et al, Cancer Research 40 (March 1980) 933-7, report a t-PA from Bowes melanoma cell. Additional information regarding the same t-PA quickly followed. See EP-A-0041766; Rijken et al, J. Biol. Chem. 256(13) (10 July 1981) 7635-41; Rijken et al, J. Biol. Chem. 257(6) (25 March 1982) 2920-5; Matsuo et al, Thrombos. Haemostas. (Stuttgart) 45(2) (1981) 225-9; Weimar et al, The Lancet (Nov. 7, 1981) 1018-20; Korninger et al, Thrombos. Haemostas. (Stuttgart) 45(3) (1981) 561-5 & 662-5; Opdenakker et al, Eur. J. Biol. (1982) 269-72; Korninger et al, J. Clin. Invest. 69 (March 1982) 573-580; Edlund et al, J. Clin. Invest. 80 (Jan. 1983) 349-352; Gronow et al, Trends in Biotechnology 1(1) (1983) 26-29; Pennica et al, Nature 301 (20 Jan. 1983) 214-21; and Bergmann et al, Science 220 (10 June 1983).

EP-A-0093619 (hereinafter referred to as the "Genentech Application") discloses (i) E. coli K12 strain 294, ATCC No. 31446, which is a transformant having a recombinant DNA compound encoding for a t-PA; (ii) E. coli K12 strain W3110 transformed by rDNA, ATCC No. 27325, from which extracts of a t-PA are prepared for the assay of fibrinolytic activity, i.e. a recombinant DNA compound; and (iii) $DHFR^+$ CHO-KI (ATCC CL 61) cells transformed for amplification, also expressing a t-PA, viz. a rDNA nucleotide encoding for a t-PA.

The given deposits are not needed to obtain cDNA encoding for t-PA of the present invention. The following description discloses a gene isolation process, from among the processes known in the art, to be applied to the Bowes melanoma cell, for obtaining the recombinant DNA compound encoding for a t-PA. The Bowes melanoma cell is commonly available to the public.

All of the above noted disclosures to rDNA encoding for a t-PA provide cDNA useful to prepare the novel t-PA of the present invention. The novel t-PA of the present invention is characterized by a difference in glycosylation from t-PA previously disclosed in the above noted references. That is, the nature and extent of glycosylation of the t-PA of the present invention is different from t-PA known heretofore; either t-PA prepared by recombinant techniques or t-PA in any naturally occurring form.

t-PA is a complex protein which has a large number of cysteine residues. Some of these residues are involved in the formation of specific disulphide bridges perhaps forming parts of complex domains of the protein. This means that for t-PA to be active it must fold properly. Experience with high level expression of some mammalian cells in E. coli has shown that the cytoplasmic environment is too reducing for these proteins to fold properly. See Cabilly, S. et al., Proc. Nat'l. Acad. Sci.:USA, vol. 81, pp. 3273-77 (1984). Thus, although the references above provide examples of t-PA there is no basis for finding the novel active t-PA of the present invention in these references.

The novel t-PA of the present invention provides the high activity of previously known t-PA, however, it is a product of expression in yeast. So such novel t-PA is not within the teachings of previous disclosures.

Specifically, the present invention provides the MFα1 signal sequences to produce an active novel t-PA product. Such signal sequences sometimes referred to as "pre-pro" portion are not suggested by the prior art as now found in the present invention. The MFα1 gene is available to the public by disclosures of Singh A. et al., "Saccharomyces Cerevisiae Contains Two Discrete Genes Coding for the α-factor Pheromone", Nucleic Acids Research, vol. 11, No. 12, pp. 4049-63 (1983); and of Kurjan, J. et al., "Structure of a Yeast Pheromone Gene (MFα): A Putative α-Factor Precursor Contains Four Tandem Copies of Mature α-Factor", Cell, vol. 30, pp. 933-43 (1982). Its uses are disclosed by Merryweather, J. P. et al., "α-Factor Promoter and Processing Signals Direct Regulated Synthesis and Secretion of Authentic Heterologous Proteins". and Singh A. et al. "α-Factor Gene and the Synthesis and Secretion of Mammalian

Proteins in Yeast" in Abstracts of the August, 1983 meeting on Molecular Biology of Yeast. Singh et al. suggested fusion of α-1230 bp DNA fragment, that contains 960 bp of MFα1 6'-flanking DNA and the sequence coding for the "pre-pro" portion of the α-factor to the mammalian genes for human interferons α1 and γ, bovine interferons α1 and $\beta_2$, HSA, or IGF. But no suggestion is found which makes obvious the MFα1 signal sequences of the present invention. In fact, no previous disclosure provides an enabling method for the expression of active t-PA in yeast. It is now found, that t-PA activity of the present invention is detected only from the yeast cells transformed with plasmids carrying the t-PA gene and the activity is plasminogen dependent.

Transformation of yeast protoplasts was first accomplished by Hinnen et al., Proc. Natl. Acad. Sc. USA, vol. 75, 1929-1933. However, due to a lack of detail in the published literature, the present invention now shows the transformation of yeast with high efficiency. Particularly, the present invention allows movement of plasmid DNA from E. coli to yeast using crude DNA preparations. Such high efficiency of transformation permits cloning by direct selection for a marker in yeast instead of cloning in E. coli first.

Generally, the nomenclature and appropriate general laboratory procedures required in this application can be found in Maniatis, T. et al., Molecular Cloning a Laboratory Manual, Cold Spring Harbor Laboratory, Cold Spring Harbor, New York, 1982.

## SUMMARY OF THE INVENTION

The present invention provides novel t-PA, recombinant DNA compounds, and yeast transformed by rDNA compounds. Additionally, the present invention provides methods for the preparation of the novel t-PA, rDNA compounds and yeast transformed by rDNA compounds. The rDNA compounds comprising segments of DNA. For example, the rDNA compounds may be nucleotides in the form of a cassette which provides a promoter, "pre-pro" or signal sequences, or in the alternative, a cassette which provides a replication origin, a selectable marker and a yeast gene 3'-end useful in the construction of either a shuttle vector or expression vector for yeast. Additionally, the rDNA compounds may be the vectors. The methods relate to a yeast transformed to produce t-PA. The rDNA compounds may have nucleotides containing sequences from E. coli or

be prepared without such nucleotides by appropriate restriction and then ligation as required to complete plasmid formation. Thus, the plasmid prepared in this way is a vector solely for use in yeast.

The active t-PA expressed by yeast of the present invention is found intracellular, i.e., within the cytoplasm and either in the cell wall, i.e., "periplasmic space" or in the extracellular fluid. However, it is not yet known whether the active t-PA material found in the extra-cellular fluid is excreted by the yeast or results from lysis of some of the cells.

Further, the invention involves DNA compounds from the selection of nucleotide sequences above and plasmids and vectors not hithertoknown for expression solely in yeast. Greater stability of the vectors for expressing t-PA in yeast can be assured by means to provide a plasmid of yeast origin only. Finally, the invention includes yeast transformed for expression of t-PA.

It will be appreciated from the disclosure to follow, the rDNA compounds comprising nucleotides encoding for t-PA of the invention can be put in E. coli and produced in large amounts. Similar procedures can be used in yeast to amplify the rDNA compounds. Transformations of yeast provides expression of novel t-PA. The novel t-PA is useful as produced in the yeast host. Appropriate derivatization or modification of t-PA genes may be used herein to provide analogs for similar use in compositions. Methods for use of novel t-PA or its analogs may be as provided by the Genentech Applications.

It will be appreciated from the foregoing that a basic aspect of this invention is the provision of a rDNA compound which is characterized by a pre-pro segment from an MFα1 gene and a gene encoding for t-PA for use in cloning in bacteria or yeast and expression in yeast. Thus, the invention includes the rDNA compound comprising:

(A) Nucleotides whose sequence comprises the "pre-pro" portion of an MFα1 gene and a segment encoding for expression of human tissue plasminogen (t-PA) or analog thereof;

(B) Nucleotides whose sequence comprises a promoter, the "pre-pro" signal sequences of an MFα1 gene, a yeast selectable marker, a 2μ yeast plasmid fragment consisting of replication origin and <u>cis</u>-acting sequence consisting of REPIII and the 3'-end of the yeast ADHI

gene;

(C) Nucleotides whose sequence consists essentially of base pairs encoding for a portion of t-PA wherein the sequence is the fragment of a plasmid having the structure pTPAHI with HindIII/SStI terminal sites;

(D) Nucleotides whose sequence consists essentially of base pairs encoding for a portion of t-PA wherein the sequence is the fragment of a plasmid having the structure pTPAHI with a SStI/Sau3A terminal sites;

(E) a plasmid comprising (1) the nucleotides whose sequence contains one or more of a yeast promoter and the "pre-pro" signal sequences of the MFα1 gene, a fragment of the 2μ yeast plasmid consisting of REPIII and replication origin, a yeast selectable marker, and a fragment consisting of the 3'-end of the yeast ADHI gene; and (2) a nucleotide whose sequence consists essentially of base pairs encoding for t-PA or an analog thereof as described hereinafter; and further the invention includes

(F) Yeast transformed with a plasmid comprising nucleotides whose sequence contains (1) a yeast promoter, (2) the "pre-pro" signal sequences of the MFα1 gene, (3) a fragment of the 2μ yeast plasmid consisting of REPIII and replication origin, (4) a yeast selectable marker, (5) a fragment consisting of the 3'-end of the yeast ADHI gene; and (6) a fragment consisting essentially of base pairs encoding for t-PA or an analog thereof.

(G) t-PA or an analog thereof having selected glycosylation;

(H) t-PA or an analog thereof when expressed by yeast;

(I) t-PA or an analog thereof when expressed by yeast transformed by a plasmid comprising (1) a yeast promoter and the "pre-pro" signal sequences of the MFα1 gene, (2) a fragment of the 2μ yeast plasmid consisting of REPIII and replication origin (3) a yeast selectable marker, (4) a fragment consisting of the 3'-end of the yeast ADHI gene; and (5) a DNA sequence consisting essentially of base pairs encoding for t-PA or an analog thereof.

More particularly the invention includes a circular recombinant DNA compound of formula XXX

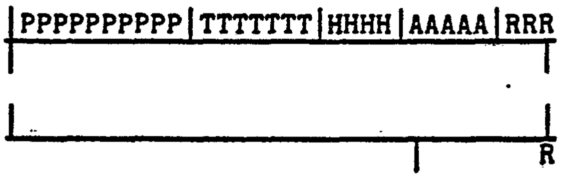


(XXX)

wherein the repeated alphabetic sequences (the number of repetitions of which is arbitrarily depicted in said formula) therein represent the following:

the "p" region comprises nucleotides whose sequences contains a yeast promoter and "pre-pro" signals consisting essentially of the "pre-pro" signals from the MFα1 gene;

the "T" region comprises nucleotides whose sequence contains DNA for the synthesis of t-PA;

the "H" region comprises the nucleotide whose sequence contains the 3'-end of the ADHI gene;

the "A" region comprises the nucleotides whose ordered codons contain the URA 3 gene;

the "R" region comprises nucleotides whose sequence contains a REPIII and replication origin from the 2μ yeast plasmid.

Each of the above described compounds of the invention may also include the nucleotides containing a sequence from the <u>E. coli</u> plasmid pBR322 that carries the kanamycin or ampicillin resistance gene ($Ap^r$) and a replication origin.

Advantageously, the pα1-TPA plasmid may be cut at XbaI and SalI sites, then ligated to produce a plasmid consisting essentially of yeast sequences in addition to the gene encoding for t-PA.

The t-PA gene from previous disclosures or as prepared in the following material may be used in the present invention. It is preferred to modify the 3' untranslated by removing some or all of about 250 nucleotides. However, such modification is not required.

The preferred promoter of the present invention is the promoter of the MFα1 gene, however, other promoters which may be used are alcohol dehydrogenaseI (ADHI), pyruvate kinase (PYK), triosphosphate isomerase (TPI), invertase gene (SUC1), and the like.

The preferred yeast selectable marker of the present invention is URA3 but other markers which may be used are <u>HIS3</u>, <u>TRPI</u>, <u>LEU2</u>,

and the like.

The preferred yeast to be used as the host cell in the present invention is Saccharomyces cerevisiae.

The t-PA or analogs thereof of the present invention may be in a composition in combination with a pharmaceutically acceptable carrier.

Use of t-PA or analogs thereof prepared by the present invention is for the treatment of thrombolytic diseases in humans. The dosage form, unit dosage and dosage ranges are within that taught in the above cited references or are within the reasonable skill of those treating the diseases.

Generally, the process of the present invention may be accomplished as follows:

A compound, either as disclosed in the references cited above or as disclosed in the present application, having an rDNA gene encoding for t-PA which may be referred to as a plasmid is generally cut at the BglII and BalI sites to excise sequences for use in the further processes of the present invention. See Chart 1 and following discussion therefor for a specific example. However, although the preferred cut at the 3' untranslated end of the t-PA gene according to the present process is BalI, the cut further and preferred modification at the 3'-end as disclosed hereinafter is not critical.

In Chart 1, TTTTTT denotes the essential region of the gene coding for the protein t-PA and UUUU shows the 3' untranslated region which is essentially excised following the initial BalI cut by manipulations in which the cut is ligated with SalI linkers and then cut with BglII. The remaining manipulations wherein the pTPA SalI has its ends partially filled and so on are merely to prepare cutting sites compatible with a yeast plasmid prepared later.

More or less, the 3' untranslated end may remain as part of the compound to be used to transform yeast for t-PA expression. For example, the cut at the 3'-end may also be made at the BglII site.

Construction of the plasmid PGG400 is accomplished in a manner within the skill of the ordinary artisan so the well known and readily available pBR322 is changed to carry kanamycin resistance rather than tetracycline resistance. Kanamycin resistance gene is readily available. (See plasmid pmL21 of the present disclosure or the Maniatis et al. refer-

ence above). Again, a specific example within the discussion is shown in Chart 5. Particularly note the showing of $Km^r$ denoting kanamycin resistance and $T_c{}^r$ denoting tetracycline resistance. Variations of the plasmid PGG400 construction may be within the present disclosure if the essential addition of kanamycin resistance to pBR322 is accomplished.

The above prepared plasmid PGG400 may be cut in a manner shown in Chart 6 and specific discussions hereinafter. The sites of the cuts are chosen because the XhoI sites may be filled to provide a site which is compatible for ligation to HincII. Of course, the cut at PuvII site produces a normally blunt end. Such a PvuII site ligates to the filled BamHI site. It can be readily seen that the cuts allow for a minimum of manipulations with the added advantage of the selection for a preferred orientation of the fragment having the ends HincII and BamHI. Such selection allows later upstream additions of rDNA having the proper orientation relative to the HincII/BamHI fragment.

The HincII/BamHI fragment is the 3'-end of the ADHI gene of the pADHBC which is publicly available or can be made by procedures of a later cited reference herein.

Other cuts in the PGG400 and pADHBC plasmid may be made by other enzymes at other sites but such cuts would require more manipulation both in the procedure shown in Chart 6 and in later procedures adding rDNA. However, alternative cuts producing the desired result may require more manipulations than shown in the examples here.

Now, generally Chart 4 shows a specific process for preparing a novel intermediate compound; i.e., plasmid pYRep31B of the present invention. This chart shows the preferred cuts to provide HindIII sites both which excise th complete URA3 gene denoted as AAAAAA and a fragment denoted by RRRRRR spanning the REPIII and replication origin and which provide the HindIII ends needed for later rDNA insertion of the AAAARRR sequence into a compound useful as a plasmid for expressing the novel t-PA in yeast.

Now the product plasmids of Chart 6 and Chart 4 are used to prepare a compound wherein the rDNA fragment having the AAAARRR sequence is inserted into a plasmid having an rDNA sequence having the ADHI 3'-end. Although the RRR fragment is not specifically shown in Chart

7 the fragment is within the cuts of the process described. The SphI cut of ($XI_2$) is selected because it is easier to eliminate HindIII base pairs by modification. Alternate sites for cutting the pYRep31B plasmid may be used, however, the cuts at the HindIII site are preferred to avoid bringing unnecessary pBR322 sequences into the product plasmid.

At this time, the process of the present invention inserts the EcoR1. HindIII portion of the MFα1 gene in Chart 8 into the plasmid prepared in Chart 7. The MFα1 gene is well known and is readily available to the public or can be obtained from pα1 as found in a site in the subsequent description of Chart 7. The HindIII sites resulting from the cuts in Chart 8 are critical in the present preferred process and although the EcoRI site also shown in cuts of Chart 8 is preferred variations of this cut are possible. For example, a cut at the XbaI site may be made instead of the cut at EcoRI, however, such a site requires filled ends and further modification making regeneration necessary after manipulation.

Finally, the cuts providing HindIII sites of plasmid pα1-ADHt are critical because of the construction of pα1-ADHt. The filled ends of the XhoI site may or may not be critical but these are preferred to obtain the final ligation to obtain the plasmid which can express in yeast the novel t-PA of the present invention.

Processes to allow variations of the promoter in combination with the pre-pro signals of the MFα1 gene although both shown as PPPPPPPP in Chart 9 are suggested in the specific embodiment.

The preferred sequences of the "pre-pro" signals from the MFα1 gene are as described in the following preferred embodiments. However, alteration of the "pre-pro" signals are possible.

All starting material for the practice of the present invention are available or can be made by known processes.

Variations within the skill of an artisan which provide processes to prepare the above noted novel t-PA or rDNA compounds and yeast having rDNA compounds therein are also the present invention.

DESCRIPTION OF THE PREFERRED EMBODIMENTS

Although the following description provides the most preferred practice of the invention it is not meant to be limiting.

Conventions used in representations of plasmids in the present

invention, though peculiar to the present application, are merely representations meant to be synonymous with conventional representations. Because plasmids may be said to be circular DNA, for convenience they are represented in the literature as circles. The present specification employs an alternative representation of plasmids as rectangles whose horizontal sides represent DNA nucleotides of the plasmid and whose two vertical sides each represent a regular phosphodiester bond which links together the bridging nucleotides represented on the horizontal sides. Further, the directional arrows which are parallel to the horizontal sides indicate the transcription for the portion of the plasmid so represented. In reading the rectangular representations of plasmids compared to a circular representation, the left side of the rectangle most conveniently will correspond to the uppermost point on the circle. Thus, the right side of the rectangle will correspond to the lowermost point on the circle. Vertical lines on either of the horizontal sides of the rectangular representation correspond to cleavage sites for restriction endonucleases or the location of the initiation or termination of a specified nucleotide sequence. Such sites are labelled in the conventional fashion. The nucleotide sequences may also be further characterized by arbitrary alphabetical characters which appear above the uppermost horizontal side and below the lowermost horizontal sides. The alphabetical characters are then appropriately defined at the bottom of each chart. One advantage of the system for representing plasmids as utilized herein is that linear segments of DNA are depicted exactly as for the circular plasmids except that one or both of the vertical sides of the rectangle are deleted. When both vertical sides are omitted, the DNA will be represented by a single horizontal line whose direction of replication may be separately indicated by horizontal directional arrows.

## DESCRIPTION OF THE SPECIFIC EMBODIMENTS

### I. EXPRESSION OF HUMAN TISSUE PLASMINOGEN ACTIVATOR (t-PA) IN THE YEAST *SACCHAROMYCES CEREVISIAE*.

#### A. MATERIALS AND METHODS

##### 1. Yeast strains and growth media:

The yeast strain YNN27 (α, *trp*1-289, *ura*3-52, *gal*2) which can be obtained from Stanford University is used for most of the work.

Yeast minimal medium (YMM) consists of 0.67% Bacto-yeast nitrogen base without amino acids and 2% glucose. The medium is supplemented with tryptophan 50-100 µg/ml for YNN27 and with histidine for LL20. YNN27 carrying some of the plasmids is grown on minimal medium supplemented with tryptophan (50-100 µg/ml) and casamino acids at 0.5%. This medium is called YMMCA. For growth on plates, agar is added to the medium to 2%.

Most of the plasmid constructions are done and propagated in the E. coli strains HB101 and MC1061.

2. Manipulations of the t-PA gene:

The t-PA gene as disclosed in EP application 0041766 or as described hereinafter is assembled in the plasmid p-TPAHI which is a starting material in the present invention (see Chart 1). Specificially, pTPA SalI is prepared herein as shown in Chart 1 by cutting plasmid pTPA with BalI according to suppliers (NEB) specifications in a 40 µl reaction for 6 hours. Restricted DNA is precipitated with ethanol, washed once with 70% ethanol, vacuum dried and resuspended in a 20 µl ligation mixture containing 50 mM Tris, pH 8.0, 20 mM DTT, 0.25 mM ATP, 50 µg/ml gelatin, 4 mM Spermidine, 1 unit of T4 ligase (Boehringer Mannheim) and 0.20 µg of SalI linkers. Ligation is continued for 16 hours. Ligated DNA is precipitated and used to transform HB 101. The transformants are screened by the minilysate procedure for the presence of a plasmid with a SalI site. The intermediate plasmid called pTPA SalI is purified on a CsC1 gradient. Then, however, the t-PA gene is modified to obtain the plasmid pTPAH. The modifications of the 5'-end to the t-PA gene are carried out in the plasmid pTPA SalI. The pTPA SalI DNA is restricted with BglII. First, the 5'-end BglII site (see chart 3 for a more detailed map of the terms used in discussing the modification) is modified such that when inserted in the HindIII site (made blunt with the polymerase described in Chart 1) the reading frame is in phase with that of the MFα1 "pre-pro" sequences and also the HindIII site is regenerated. Then, the approximately 250 nucleotides of the 3' untranslated region are removed to bring the yeast ADHI polyadenylation site, added as part of the plasmid used to transform yeast in a subsequent description, closer to the termination codon of the t-PA gene. It is believed the proximate relationship of the

polyadenylation and the termination codon of the t-PA gene improves the level of expression of t-PA in Saccharomyces cerevisiae.

The map of the t-PA gene in Chart 3 shows an outline of various regions. The BglII site (AGA TCT) is at the juncture of the "pre" and mature sequence regions of t-PA. The first codon in the mature region sequence is TCT (serine). Restriction of t-PA DNA with BglII will leave a sticky end GATC. If this end is partially filled by putting in bases complementary to C and T followed by removal of GA with single-strand specific nucleases like S1 or mung bean nuclease it can be blunt end ligated to a filled in HindIII site. Thus, ligation will restore the HindIII site as well as retain the correct translational phase between MFα1 and t-PA sequences. The plasmid pTPA-SalI has a HindIII site upstream from the BglII site of t-PA gene as shown in (II) of Chart 1.

Specifically, the modifications are as follows: The plasmid pTPA-SalI is cut at the BglII site. The cut DNA is incubated with $T_4$ polymerase in the presence of dGTP and dATP for 30 minutes at 37° C. DNA is precipitated and resuspended in a low pH buffer used for mung been nuclease treatment. It consists of 30 mM NaOAc (pH 4.6), 250 mM NaCl, 1mM $ZnCl_2$, 5% glycerol. The mung bean nuclease treatment used exactly as described by Rosenberg et al., in Methods in Enzymology, 101, pp. 123-130. The nuclease is inactivated by phenol extraction and the DNA is again precipitated, resuspended in HindIII restriction buffer and cut with HindIII. The sticky ends are filled in using the polymerase followed by ligation of DNA. Ligated DNA is used to transform E. coli HB101 and the plasmids are screened using HindIII as the diagnostic cut. A number of plasmids in which the HindIII site is regenerated are isolated. One of these plasmids called pTPAH is further characterized by restriction enzyme analysis.

Subsequently, the pTPAHI plasmid is used to provide a fragment which is then treated to modify the 3'-end of the t-PA gene. The desired result is to get rid of the long untranslated region at the 3'-end of the gene. First, an EcoRI-SalI fragment of pTPAH is purified which is then cut with Sau3A (shown in Chart 3). The sticky ends are filled using $T_4$ polymerase according to the above analogous procedure and the DNA is again restricted with the enzyme SStI (see Charts 2

and 9). A 389 base pair fragment is isolated on a gel. This fragment is ligated with two others to construct a vector called pα1ADHt-TPA as described in subsequent material and shown in Chart 9. The manipulation of the 3'-end of t-PA leave only 36 base pairs from the untranslated region instead of the approximately 250 present in the pTPA-SalI plasmid.

3. Construction of the Yeast Expression Vector pα1-ADHt.

i. A URA3-REPIII-ori Cassette in pYRep 31B

First, a fragment spanning the REPIII and replication origin sequences from the 2μ plasmid is cloned into the SmaI site of URA3 gene in plasmid Y1P31. The 2μ plasmid and the Y1P31 plasmid are well known and available to the public. For information on the 2μ plasmid see Broach, J. R., Molecular Biology of the Yeast Saccharomyces cerevisiae [Life Cycle and Inheritance], pp. 445-470. The REPIII region is discussed by Jayraman, M. et al., in "The Yeast Plasmid 2μ circle encodes components required for its high copy propagation", Cell, vol. 34, pp. 95-104 (1983) and also by Kikuchi, Y. in Cell, vol. 35, pp. 487-493 (1983).

This first cloning of the larger fragment is shown in Chart 4.

The next step is to position the URA3-REPIII-ori cassette in the plasmid pADHt.

ii. Construction of the plasmid pADHt

The starting plasmid for these constructions is PGG400 approximately 4.0 Kb in size. Its construction is shown in Chart 5 and is accomplished by methods well known in the art from two plasmids readily available to the public. Specifically, the gene coding for tetracycline (Tc) resistance in pBR322 is replaced by a fragment of DNA from pML21 which codes for resistance to Kanamycin (Km). pmL21 is cut with PuvII. The PuvII cut DNA when ligated to a filled EcoRI site regenerates EcoRI site after ligation. Plasmid pBR322 is cut with EcoRI and PvuII and the EcoRI site is filled wilth $T_4$ DNA polymerase. This DNA is then ligated to the PvuII cut pmL21 and transformed into E. coli followed by selection for $Km^r$ and $Ap^r$ but $Tc^s$ clones. The selection gave PGG400 which was used as the starting plasmid to construct pADHt.

First, the 3'-end of yeast ADHI (alcohol dehydrogenase I) which is identified by Bennetzen, J. L. et al., J. Biol. Chem., vol. 257, pp. 3018-3025 (1982) is inserted into a PvuII/Xho I cut PGG400 as follows. The 3'-end of the ADHI gene is inserted by first restricting

the plasmid pADHBC in a manner analogous to that of Williamson, V. M., et al., Nature, vol. 283, pp. 214-16 (1980) and Williamson, V. M. et al. Cell, vol. 23, pp. 605-14 (1981) with HincII and BamHI followed by filling the ends to obtain the insert and then by ligating with the cut PGG400. Only the end fragments when inserted in the appropriate orientation regenerate the BamHI and Xhol sites. A pADHt which carries the 3'-end of the ADHI gene is thus isolated from the putative candidates. Construction of pADHt is depicted in Chart 6. The authenticity of the cloned ADH 3'-end is confirmed by restriction enzyme analysis and by sequencing.

The pADHt is modified by changing the BamHI site to SalI. This is done by restricting the DNA with BamHI followed by insertion by ligation of an 8-mer SalI linker into the filled BamHI site.

iii. URA3-REPIII-ori Cassette in the Plasmid pADHt

A HindIII fragment (approximately 2.4 Kb) from the plasmid pYRep31B above is purified. The fragment is then inserted into the SphI site of plasmid ADHt after making the SphI site ends blunt in a manner analogous to that described above to make ends blunt. The SphI site is located in the 3'-end fragment of ADHI previously cloned in pADHt. Of the two possible orientations, the one with the clockwise transcription is isolated. The URA3-REPIII-*ori* fragment is a cassette useful in expressing t-PA in yeast. The method of positioning the URA3-REPIII-*ori* cassette in pADHt to obtain the plasmid pADHt-REPIII is shown in Chart 7.

iv. Final Construction Step to Plasmid pα1-ADHt

The construction of pα1-ADHt is completed by replacing the EcoRI-HindIII fragment in pADHt-REPIII with the EcoRI-HindIII fragment of the MFα1 gene in plasmid pα1. The plasmid pα1 can be obtained by following the instructions in the above noted references by Singh, A. et al. and Kurjan, J. et al. The EcoRI-HindIII fragment of MFα1 brings in the promoter and the signal sequences required for secretion of the t-PA expressed in yeast. The plasmid pADHt-REPIII is a useful intermediate in terms of constructing expression vectors, for example, the EcoRI-HindIII or EcoRI-SmaI fragment can be replaced with any desired DNA fragment carrying promoter sequences. Alternatively, it can provide a SalI-XhoI fragment carrying a replication origin,

a selectable marker and yeast gene 3'-end. This construction is shown in Chart 8.

The methods required by use of the intermediate plasmid pADHt-REPIII to construct expression vectors are described herein or are known to the ordinarily skilled artisan.

4. Construction of the plasmid pα1ADHt-TPA

Finally, purified fragments are prepared as follows. Plasmid pα1-ADHt is cut at the XhoI site and the ends are filled in. Then the DNA sequence of cut plasmid pα1-ADHt is cut at the HindIII site. The larger of the resulting fragment is purified. The result is shown as (III') in Chart 9.

Next, two fragments are prepared from the plasmid pTPAH. The first fragment results from cuts with HindIII/SStI. The second fragment results from cuts with Sau3A/SStI. Each of these are shown as II' and II", respectively in Chart 9. The Sau3A end is filled to make a blunt end. The fragments shown as III', II' and II" are all purified. Finally, the three purified fragments are ligated. The blunt end Sau3A ligated with filled in Xho site regenerates the XhoI site. E. coli HB101 is transformed with the ligated DNA and screened for the appropriate plasmid. pα1ADHt-TPA plasmid is thus isolated. A summary of the final procedure discussed here is shown in Chart 9.

5. Transformation of pα1-ADHt-TPA into YNN27:

Yeast cells are grown to an $O.D._{550}$-0.5-0.6 in YEPD (2% glucose, 2% peptone, 1% yeast extract) medium. Depending on the number of samples for transformation, 20 to 120 ml cultures are used. A 20 ml culture is usually sufficient for a single transformation. The culture is usually started from a fresh overnight culture grown by aeration. The dilution from the overnight culture is such that the starting $O.D._{550}$ is around 0.1 to 0.15. Usually, yeast cultures suffer a lag in growth after dilution in fresh YEPD (unadjusted pH 6.9). The lag period is minimized or eliminated by preparing YEPD with pH adjusted from 5.5 to 5.6. Under these conditions, cultures are ready for harvesting in from 4 to 4.5 hours which is equivalent to about two doublings of cells. When the culture has reached the desired O.D., cells are harvested by sedimentation in the bench-top Sorvall at room temperature. After decanting the supernatant, cells are washed

once with 20 ml 0.8-1.0 M Sorbitol solution buffered with Tris to pH 7.6. Sorbitol solutions are quite acidic and the pH varies with each batch. Many times, making the Sobitol solution 10 mM in Tris from a 1 M Tris stock solution, pH 7.6, may not bring up the pH. pH should be checked and adjusted if need be. After washing, cells are suspended in the same solution, i.e., 0.8-1.0 M Sorbitol, usually in 10-20 ml volume. For cultures up to 50 ml starting volume, the cells are suspended in 10 ml of Sorbitol and for larger cultures, 20 ml of Sorbitol is used. Mercaptoethanol is added to 10 mM followed by the addition of 390 units (15 µl) of lyticase (use 8-10 µl of enzyme if the starting culture is 50 ml or less). The cell-lyticase mixture is incubated for 20-25 minutes at 30° C if LL20 is used, or for 25-30 minutes if the strain NN27 is used. At the end of this period, cells are gently pelleted (2.5 K, 4-5 minutes) in the bench top Sorvall. The protoplasted cells are washed twice with 10 ml volumes of 0.5 M $MgCl_2$, buffered with 10 mM Tris, pH 7.4 or 7.6. Resuspension of protoplasts should be gentle. After the washes, the protoplasts are suspended in concentrated form (0.2 ml for 20 ml cultures in a mixture containing 0.5 M $MgCl_2$, 25 mM $CaCl_2$, 10 mM Tris pH 8.3) and chilled on ice. Crude plasmid (as a lysate from E. coli) pα1ADHt-TPA in 10-20 µl volume of TE is added to and mixed well with the protoplasts. After a 15 minute incubation on ice, the protoplasts are transferred to room temperature for 15 minutes. The protoplasts tend to settle to the bottom of the tube and should be resuspended once by gently rotating the tube. After the room temperature incubation, a 10-fold excess of 15% wt/wt polyethylene glycol (PEG 4000) solution, buffered with 10 mM sodium acetate (NaAc), pH 5.5, and containing 25 mM $CaCl_2$, is added to protoplasts. Protoplasts should be mixed thoroughly in PEG by rolling the tube while tilting it up and down. After a 12-15 minute exposure to PEG, protoplasts are pelleted and resuspended in a medium containing 30-50% YEPD and 0.5 M $MgCl_2$, following removal of PEG. After appropriate dilutions in the same medium, protoplasts (in 0.1 ml volume) are mixed with 8-10 ml of 3% agar containing .8-1M sorbitol, 0.7% yeast nitrogen base, pH 5.4, 1% YEPD and other ingredients (amino acids, etc.) as required, and spread on the same agar medium except that the agar concentration is 2%. Also, the pH of

the bottom agar layer is 6.5 and it does not contain 1% YEPD. If the strain has other auxotrophic requirements, i.e., requirements other than the selectable marker, the appropriate ingredients should be added at 2.5 times the amount normally required by intact yeast cells. Plates are incubated at 30° C for 2-4 days.

It should be mentioned that part of the problem in protoplast transformation is the shear mechanics of handling the protoplasts and other materials. For example, 3% agar is difficult to transfer around. Normally, it is advised to prepare the melted agar and distribute it to tubes which are kept at 50° C in a water bath. Preferred for small amounts such as handled here it is better to add the protoplasts to the agar kept at 50° C and then mix by rotating the tube between both hands and spread quickly on a plate.

Using the above protocol, transformants of pα1ADHt-TPA are obtained. Due to the number of steps and other variables, there is considerable variation in the results from experiment to experiment. However, the procedure is reasonably reproducible. A method by which yeast cells can be transformed without making protoplasts has recently been developed by a Japanese group (see Kurjan, J. et al. (1982) Cell, vol. 30, pp. 933-943). This method of transforming yeast cells is also contemplated by the present invention for obtaining transformants of pα1ADHt-TPA.

6. Assay for t-PA activity:

t-PA acitivity in yeast extracts and in the growth medium is monitored by measuring the cleavage of the chromogenic substrate H-DVal-leu-lys-NH-4-nitrocyclohexane (Halena Laboratories) by plasmin. The reaction mixture (~2 ml) consists of 1.7 ml Tris buffer, pH 7.5, 100 μl of plasminogen (stock solution in 1 CU unit/ml), 100 μl of 6 x $10^{-3}$ M substrate stock solution, and 50-100 μl of sample to be assayed for t-PA activity. The reaction is monitored at 37° C over a 400-600 minute period at 405 nm in a Beckman DU-7 spectrophotometer.

7. Preparation of yeast extracts:

Yeast cell extracts for measuring t-PA activity are prepared as follows.

Cells are grown in 20-100 ml of YMM or YMMCA medium supplemented with the appropriate nutrients. The growth period ranges from 4 to

24 hours. After harvesting the cells with a spin in the Sorvall centrifuge, the cell pellet is suspended in ~1 ml of 10 mM Tris buffer, pH 7.5. Glass beads (0.5 mm in diameter) are added to the cells until the level is ~1 mm below the meniscus. Cells are lysed by vortexing over five one-minute intervals. Cells are chilled for one minute on ice after every vortexing interval. Approximately 0.5 to 1.0 ml samples are taken out and cleared of the cell debris by centrifuging in the Eppendorf centrifuge for one minute. Samples, if not immediately used, are stored frozen at -20° C.

t-PA activity in the growth medium is monitored after spinning out the cells by a high speed centrifugation.

B. RESULTS

1. Expression of active t-PA in yeast:

One of the first objectives in the assay for t-PA activity is to see whether the yeast cells produced any t-PA activity when transformed with the plasmid pα1ADHt-TPA. A number of criteria have to be satisfied for establishing the authenticity of the t-PA activity produced in yeast. For example, it has to be ascertained that there is no endogenous plasmin-like activity in yeast that would cleave the substrate or a t-PA-like activity that would activate plasminogen. In order to investigate all these possibilities an experiment is set up in which t-PA activity is monitored in the cell extracts in the presence or absence of plasminogen. Thus, assays were set up as follows:

(A) Cell extracts from YNN27 (no plasmid) + substrate and with no plasminogen.

(B) Cell extracts from YNN27 (no plasmid) + substrate + plasminogen.

(C) Cell extracts from YNN27 (pα1ADHt-TPA) + substrate and with no plasminogen.

(D) Cell extracts from YNN27 (pα1ADHt-TPA) + substrate + plasminogen.

(E) Extracellular fluid from YNN27 (pα1ADHt-TPA) culture + substrate + plasminogen.

Results are consistent with a finding that a) there are no endogenous t-PA-like plasminogen dependent or plasmin-like activities in yeast, and b) t-PA activity is detected only in the extracts prepared from cells harboring the t-PA gene containing plasmid pα1ADHt-TPA.

Experiments analyzing the time course of t-PA synthesis are now

conducted. A 120 ml YMMCA culture of YNN27 (pα1ADHt-TPA) is started by dilution of an overnight culture. Initial $O.D._{550}$ is ≈0.2. The growth medium is supplemented with 100 μg/ml of tryptophan. Twenty ml samples are withdrawn at 3 hour intervals. Extracellular fluid and cell extracts are monitored for t-PA activity as described above in "A. Materials and Methods." Assay results of cell extracts clearly show that the rate of t-PA synthesis is about the same in the first 9 hours of growth if allowance is made for lower number of cells at earlier times. The highest amount of t-PA synthesis is between 9 and 12 hours. After that time, there is a drop in the amount of t-PA activity that is found in cell extracts. After 26 hours of growth, the amount of t-PA activity drops to a very low level. This is presumably due to accelerated proteolytic degradation as the cells enter the stationary phase. The assay results for t-PA activity in extracellular fluid show a much lower level. However, the assaying of extracellular fluid is for the activity in 100 μl volume of a 20 ml sample, whereas the results of assaying of cell extracts are from a concentrated form. The activity in extracellular fluid is the highest in the 3 hour sample.

## II. ISOLATION OF A FULL LENGTH HUMAN TISSUE PLASMINOGEN ACTIVATOR cDNA (t-PA cDNA)

### A. MATERIALS AND METHODS

#### 1. Growth of Bowes Melanoma Cells

Bowes represents an established cell line derived from a human spontaneous melanoma and is a gift of Dr. Daniel Rifkin of the New York University School of Medicine. Cells are cultured in Dulbecco's Modified Eagle Medium (Gibco) supplemented with 10% fetal bovine serum (Gibco) and 50 μg/ml gentamycin (Sigma) in a humidified atmosphere at 37°, using 95% air/5% $CO_2$. Cells harvested for RNA preparation are grown to confluency in Falcon 150 $cm^2$ flasks.

#### 2. RNA Preparation

RNA from Bowes melanoma cells is isolated essentially as described by Lizardi and Engelberg, and described herein on a per-flask (15 $cm^2$) basis. See Lazardi, et al. Anal. Biochem., vol. 98, pp. 116-122 (1979). Cells are washed twice with 25 ml of ice cold PBS and subsequently collected and lysed in 6 ml lysis buffer (2.4% SDS, 0.1 M NaCl; 7.5 mM EDTA; 20 μg/ml polyvinyl sulfate; 25 mM Tris base pH

7.35). Flasks are rinsed with an additional 2.5 ml of lysis buffer, and the lysates are pooled. Sodium perchlorate is added to 1.4 M and the lysate is incubated at 50° C, agitating until clear (~4 minutes). The viscosity of the solution is reduced by passing it twice through a 20 gauge needle. Nucleic acids are precipitated with 4 volumes of EPR ($NaClO_4$-saturated ethanol/water) and left at 4° for 60 minutes. The precipitate is collected by centrifugation in a Sorvall Superspeed centrifuge at 5,000 RPM for 15 minutes at 4°. The pellet is dissolved in 15 ml of a solution consisting of 25 mM Tris, pH 7.35; 0.2% SDS; 1 mM EDTA; 0.2 M NaCl and nucleic acids are reprecipitated by adding 0.6 volumes of isopropanol and incubating at -20° for 4 hours. The precipitate is collected as above, the pellet is dissolved in sterile distilled $H_2O$ (2.5 ml per 150 $cm^2$ flask) NaCl is added to 0.3 M and the nucleic acids are precipitated with 2 volumes of ethanol overnight at -20° C. The precipitate is again collected as described and the pellet is dissolved in 10 mM Tris pH 7.4; 1 mM EDTA; 0.1 M NaCl and 0.1% SDS, phenol extracted, and EtOH precipitated. The nucleic acid pellet is dissolved to approximately 1 mg/ml in DNase buffer (10 mM MES (Sigma) pH 5.5; 0.1 M NaCl; 5 mM $MgCl_2$; 10 µg/ml polyvinyl sulfate), RNase free DNase (Miles) is added to 1.5 µg/ml and the solution is incubated at 30° for 20 minutes. Proteins are removed by extraction with an equal volume of a phenol:chloroform:isoamyl alcohol mixture (PCl is an equal volume of phenol added to a 24:1 mixture of chloroform:isoamyl alcohol). Phases are separated by centrifugation and residual phenol is removed from the aqueous phase by extraction with an equal volume of chloroform:isoamyl alcohol (Cl-24:1). Again the phases are separated and the RNA is ethanol precipitated. A typical yield from 10-150 $cm^2$ flasks grown to confluency is about 8-10 mg total RNA.

3. OdT Selection of polyA + mRNA

RNA is polyA + enriched by passage over an oligodeoxythymidine-cellulose (OdT-cellulose-Collaborative Research) column as described by Aviv-Leder (see Aviv, H. et al., Proc. Nat. Acad. Sci., vol. 69, pp. 1408-12 (1972)). Briefly, 0.1 g of OdT-Cellulose is packed in a 5 ml syringe and swelled with sterile, distilled $H_2O$. The column is equilibrated with ~ 10 column volumes of 10 mM Tris buffer, pH

7.4; 1 mM EDTA; 0.5% SDS; 0.5 M NaCl (column equilibration buffer - CEB). RNA is dissolved in 1-2 ml of 10 mM Tris base pH 7.4; 1 mM EDTA and 0.5% SDS, heated to 65° for 5 minutes, and diluted to 10 ml in CEB, bringing the final NaCl concentration to 0.5 M. The dilute RNA solution is passed over the OdT-cellulose several times to assure maximum binding of polyA mRNA. Nonspecifically bound RNA is washed off with fresh CEB (10-20 column volumes) and the polyA + RNA is eluted in 4 ml sterile distilled $H_2O$. NaCl is added to 0.3 M, 2 volumes of ethanol is added and the polyA + mRNA is precipitated by incubation in a dry-ice-ethanol bath for 60 minutes. The precipitate is collected, dissolved in 0.5 ml sterile distilled $H_2O$, Cl extracted, and reprecipitated all as described above. Typical yield from 10-150 $cm^2$ flasks twice selected on separated OdT columns is ~ 50 μg polyA + mRNA.

4. Sucrose Gradient Fractionation of polyA + mRNA

The polyA + mRNA is fractionated by centrifugation through 15-30% linear sucrose gradients prepared as follows. Sucrose (ultrapure, BRL), is dissolved to either 15%, or 30% in 50 mM Tris base pH 7.4; 0.1 M NaCl; 0.1% SDS and 1 mM EDTA and deposited in a BioRad plexiglass conical gradient maker connected to a Buchler Auto Densi-Flow Model IIC peristaltic pump which allowed formation of the gradient from bottom to top (30%-15%). PolyA + mRNA (~300 μg) dissolved in 200-400 μl of sterile distilled $H_2O$ is loaded onto the gradient and centrifuged in a Beckman SW41 Ti rotor at 35,000 RPM for 18 hours, 0.5 ml fractions are collected, again using the Buchler Auto Densi-Flow Model IIC to harvest from top to bottom, connnected to a Gilson Micro fractionator (Model FC80-K).

Aliquots from each fraction is injected into Xenopus oocytes and translation products are assayed for plasminogen activator activity by the casein-agar plate method analogous to that described by Miskin, R. et al., Nuc. Acids Res. vol. 9, pp. 3355-63.

Specifically, the tissue plasminogen activator t-PA is assayed by observing the conversion of plasminogen to plasmin, a non-specific protease, which digests casein. The assay is performed as follows. 0.5 ml of 8% casein, boiled, is mixed with 1 ml 2 x MBS and heated to 50° C. 3% agarose is melted and cooled to 50° C. 0.5 ml of the 3% agarose is added to the casein/MBS and mixed by pipetting. 100

μl of 1 mg/ml human plasminogen is added (final concentration is 50 μg/ml) with mixing and the material is poured into a 3.5 cm diameter plastic petri dish. The agarose solution is cooled to room temperature (about 30 minutes). 2 mm diameter holes in the agarose are cut with Biorad cutter gel punches. The holes are made just before use and the wells are filled with MBS. One or two oocytes which have been injected with PA RNA at least 6 hours prior to assay are added to each well. Incubation is in a humidified dish for 12-24 hours. Clear zones of casein hydrolysis are readily observed.

Fractions enriched for t-PA mRNA are precipitated by addition of 2 volumes of ethanol and incubating on dry ice for 1 hour. Precipitates are collected by centrifugation at room temperature in an Eppendorf table-top microfuge, dissolved in sterile, distilled $H_2O$, pooled, and reprecipitated. This t-PA enriched polyA + mRNA is used to generate cDNA for cloning.

5. cDNA Synthesis

All of these reactions are assembled on ice.

(A) First Strand Synthesis using $OdT_{12-18}$ Primer

10 μg of enriched, polyA + selected Bowes mRNA (in μl $H_2O$) is incubated at room temperature for 10' in the presence of 10 mM MeHgOH (Alfa) to aid in the unfolding of secondary structure. MeHgOH is highly toxic and should be handled in a fume hood. Subsequently, the following components are added; β-mercaptoethanol to 28 mM; RNasin (Biotec Inc) 1 unit/μl final reaction vol; 75 mM Tris base pH 8.3; 6 mM $MgCl_2$; 50 mM KCl; 10 μg oligodeoxythymidine ($OdT_{12-18}$-Collaborative Research); 100 μCi α-$^{32}$p-dCTP (Amersham); dGTP, dTTP, and dATP to 500 μm each; dCTP to 250 μm (all nucleotides from P. L. Biochemicals, dissolved to 20 mM in 10 mM Tris base pH 8 and neutralized with 1.0 M NaOH) and reverse transcriptase (Life Sciences Inc) 1 unit/μg input RNA, in a final reaction volume of 50 μl. Prior to incubation, 1 μul is removed and spotted onto a nitrocellulose filter (Schleicher and Schuell). After 60' at 42°, 1 μl is again removed and spotted on a separate filter. The reaction is terminated by the addition of EDTA to 10 mM, and after phenol extraction, the cDNA is precipitated as described above. The filters are washed extensively (4 x 200 ml) in ice cold 5% trichloroacetic acid (TCA), dried, and dissolved in

Liquifluor scintillation fluid (New England Nuclear). TCA precipitable radioactivity is determined in a Beckman LS9000 scintillation counter. The difference between the 0 and 60 minute time points represents the net incorporation of dCTP from which the mass yield of cDNA may be calculated according to the formula:

$$\text{Ci incorporated} \times \text{specific activity (mmole dCTP/Ci) of reaction} \times \frac{\text{mass dCTP}}{\text{mmole dCTP}} \times 4 \text{ nucleotides} = \text{mass of single stranded cDNA}$$

Mass conversion of RNA to cDNA using oligod$T_{12-18}$ as a primer has varied between 15-40%, the typical conversion being ~30%.

(B) First Strand Synthesis using Specific Pentadecamers as Primers (see Panabieres, F. et al., Gene, vol. 19, pp. 321-6 (1982) for generally analogous procedures).

Twenty-Five µg of polyA + selected RNA is incubated with 1 µg of primer (5-10-fold picomolar excess of primer to template) and 1.5 mM EDTA in 58 µl at 90° C. The mixture is allowed to equilibrate to room temperature slowly by immersion in a 5 ml water bath heated initially to 90°. After annealing the pentadecamer to the RNA template the following reagents are added: dithiothreitol (DTT) to 2 mM; RNasin to 1 unit/µl final reaction volume; 100 mM Tris base, pH 8.3; 11 mM $MgCl_2$; 50 mM KCl; 100 µCi $\alpha$-$^{32}$P-dCTP; 500 µm each of dGTP, dTTP, and dATP; 250 µM dCTP; reverse transcriptase (Life Sciences, Inc.) to 1 unit/µg RNA. Incubation is at 20° C for 3 hours at which point an equal amount of additional reverse transcriptase is added and the reaction is allowed to continue at 50° for 30 minutes. Aliquots are withdrawn at 0, 180 minutes and 210 minutes for determination of incorporation as described above. Typical mass conversions when synthesizing specific cDNA by primer extension are 5 to 6% using Bowes polyA + selected mRNA and t-PA specific oligonucleotides.

(C) Removal of the RNA Template

EDTA is added to 10 mM and the RNA template is degraded by the addition of NaOH to 0.3 M (from freshly prepared 3 M stock). Incubation is at 42° for 60'. The reaction is neutralized by addition of HCl to 0.3 M after which it is extracted with an equal volume of PCl. Phases are separated by centrifugation at room temperature in an Eppendorf microfuge, the upper aqueous phase is removed and the interface reextracted

2X with 1/2 volume of 1 mM EDTA, 0.1 M NaCl; 0.1% SDS and 10 mM Tris base pH 7.5 (NETS). The aqueous phases are pooled and extracted with PCl. Again, the upper aqueous phases are removed and the interface reextracted 1X with 1 volume NETS buffer. NaCl is added to 0.3 M, 2 volumes of ethanol are added and the single stranded cDNA is precipitated by incubating on dry ice for 30-60'. The precipitate is collected by centrifugation in an Eppendorf table top microfuge for 15' at room temperature. The pellet is dissolved in 200 µl sterile distilled $H_2O$ adjusted to 0.3 M NaCl, and ethanol-precipitated again as described. After pelleting, the cDNA is briefly dried in a Savant Speed Vac Concentrator.

(D) Second Strand Synthesis

The second strand is synthesized in a reaction consisting of 40 mM $KPO_4$ (K-phosphate) pH 7.5; 6.6 mM Mf of $Cl_2$; 1 mM DTT; 500 µm each of dGTP, dTTP, dATP; 250 µm dCTP; 100 µCi $^3H$-dCTP (Amersham) and 1 unit/µg input RNA of DNA polymerase Klenow fragment (BRL) in a final volume of 100 µl. The reaction is incubated at 15° C for 4 hours and incorporation of $^3H$-dCTP is followed as described for $\alpha$-$^{32}P$-dCTP. The reaction is terminated, PCl extracted, and double stranded cDNA precipitated all as described above for first strand synthesis with the exception that $NH_4Ac$ is added to 2.5 M (rather than NaCl to 0.3 M) for the first ethanol precipitation. $NH_4Ac$ minimizes coprecipitation of unincorporated nucleotide triphosphates to which $S_1$ nuclease is sensitive.

(E) $S_1$ Removal of Hairpin Structure

$S_1$ reactions are carried out in a 50 µl reaction volume consisting of 30 mM NaAc pH 4.6; 0.3 M NaCl; 3 mM $ZnSO_4$ and $S_1$ nuclease (Miles Laboratories) at 0.25 µg $S_1$ per µg cDNA. Incubation is at 37° for 15'. Successful elimination of hairpins is determined by increased in TCA-soluble counts as previously described. The reaction is terminated by phenol extraction as described, omitting the precipitations. The aqueous phases are pooled and loaded directly onto sucrose gradients identical to those used for fractionation of polyA + mRNA. Fractions are similarly collected and assayed this time by dissolving 5 µl aliquots into 10 ml of Aquafluor scintillation fluid (New England Nuclear) and measuring both $^{32}P$ and $^3H$. cDNA from relevant fractions are precipitated by addition of 2 volumes of ethanol and incubating on dry ice for 30'. The precipitates are pelleted by centrifugation for 15' at room temperature in an Eppendorf microfuge. Pellets are dissolved in sterile 0.3 M NaCl, pooled and reprecipitated. The precipitates are pelleted and dried as described.

(F) Tailing

Homopolymer tracts of dCTP are enzymatically added to the 3' termini of the cDNA molecules in a 50 µl reaction made up (on ice) of 100 mM K Cacodylate (Sigma) pH 7.5; 2 mM $CoCl_2$; 1 mM EDTA; 50 µM dCTP; 100 µCi $\alpha$-$^{32}$P-dCTP and 12 units of deoxynucleotidyl transferase (TT-Miles). A 1 µl 0-time point is spotted onto nitrocellulose and the reaction is initiated by incubating at 12°. After 30 seconds, another 1 µl aliquot is removed. The remainder of the reaction is immediately placed on dry ice while tail length is determined. The filters containing aliquots from the 0 and 30 second time points are washed in ice cold 5% TCA, dried and counted as described above. Net incorporation yields a statistical average of residues of dCTP added which when divided by the number of 3' termini in the reaction allows the determination of tail length per end. Ideally, 10-30 residues of dCTP should be added to maximize cloning efficiency. If after 30 seconds the tail length falls short of this range, the remaining reaction volume is thawed on ice and reincubated at 12° C, titering at 15 second intervals until tails of adequate length are added. The reaction is then phenol extracted ethanol precipitated and dried as described under second strand synthesis ($NH^4Ac$ minimizes coprecipitation of $CoCl_2$).

(G) Annealing to Vector DNA

Vector DNA (prepared by digesting pBR322 to completion with PstI and adding homopolymer tracts of dGTP residues) is purchased from New England Nuclear. Tailed cDNA is mixed with vector in a 1:1 molar ratio in a 50 µl reaction containing 10 mM Tris pH 7.4; 0.4 M NaCl; 1 mM EDTA. Final DNA concentrations varied between 20-60 µg/ml. Annealing is accomplished by either; 1) following a defined regimen of incubations consisting of 65°/10'; 42°/60'; 37°/2 hours, and then room temperature for 2 hours, or 2) incubation at 65°/10' shutting off the water bath and allowing it to slowly equilibrate to room temper-

ature overnight. Both methods appear to work equally well.

In the cDNA synthesis described above various materials are used which are known or which can be synthesized by known methods. The following descriptions numbered 6. through 10. provide the synthesis or methods as noted for use in appropriate steps of the cDNA synthesis.

6. Oligonucleotide Synthesis

Three pentadecamers are used either as probes or as primers for cDNA synthesis. Their sequences are:

| | 5' | | | | 3' | complementary to 3' t-PA positions; |
|---|---|---|---|---|---|---|
| 1. | TCA | CGG | TCG | CAT | GTT | 1759-1773 |
| 2. | GGG | GTT | TGA | GTC | TCG | 634-648 |
| 3. | CCC | ATC | AGG | ATT | CCG | 886-900 |

They are synthesized and purified by methods analogous to those described in:

7. Transformations

Transformations are carried out which are analogous to those described by Clark-Curtis, G.E. and Curtis, Roy, III, <u>Methods in Enzymology</u>, vol. 101, pp. 347-62 (1983).

8. Restriction Analyses

All restriction enzyme digestions are carried out as suggested by New England Biolabs.

9. <u>In situ</u> Screening

Colony hybridization using double stranded DNA fragments as probes is carried out as described in Grunstein, M et al., Proc. Nat. Acad. Sci., 72, vol. 72, pp. 3961-5 (1975) but modified to provide the following method: Nitrocellulose filters are made which contain colonies of the cells prepared above. The filters are then laid, colony-side up, onto a bed of 5-8 thickness of Whatman 3MM paper soaked in denaturing solution consisting of 1.5 M NaCl, 0.1 M NaOH. The denaturant is allowed to diffuse upwards into the colonies for 1.5-2 minutes at which time the filters are transferred to a second bed of 3MM papers soaked in neutralizing solution containing 0.2 M Tris·HCl pH 7.4, 2 x SSC (1 x SSC = 0.15 M NaCl; 0.015 M Na Citrate; pH 7.1), and 25 mM EDTA, for 1-3 minutes. Filters are then thoroughly air dried and

baked *in vacuo* for 2-4 hours at 80° C.

Hybridization conditions are as previously described by Goeddel, D.V. et al., *Nature*, vol. 290, pp. 20-26 (1981). Filters are prehybridized batchwise in 50% formamide, 5x Denhardts reagent (1x Denhardts = 0.02% bovine serum albumin; 0.02% polyvinyl pyrrolidone, 0.02% ficol, MW-400,000), 50 mM sodium phosphate buffer pH 6.7, and 400 µg/ml denatured salmon sperm DNA at 42° C for 12 hours with moderate agitation.

The prehybridization solution is then withdrawn from the hybridization vessel and heated to 68° C. Dextran sulfate (Sigma, MW $\geq$ 500,000) is added to a final concentration of 10%. The probe is denatured, along with 20 µg of unlabelled pBR322 digested with HinfI, by boiling for 5 minutes. Both denatured DNAs are immediately diluted into, and mixed well with, the pre-heated prehybridization mix. The solution is added back to the filters and allowed to hybridize at 42° for 16 hours with moderate agitation.

After hybridization, the probe is removed and saved, and the filters are washed in 0.1% SDS, 0.2x SSC for a total of 3 hours with 5 changes of 400 ml each. Filters are thoroughly air dried, mounted, and autoradiographed using Kodak X-OMAT AR film and Dupont Cronex Lightnening Plus intensifying screens for 16 hours at -70° C.

The colony hybridizations described here use the pentadecamers also described above. For this use, in order to obtain maximum efficiency during assembly of the primer repaired fragment with its 3' counterpart and vector, it is necessary to phosphorylate the 5' terminus of the primer. 1 µg of 15-mer is phosphorylated in a 50 µl reaction volume consisting of 70 mM Tris-base (pH 7.6), 100 mM KCl; 10 mM $MgCl_2$, 5 mM dithiothreitol; 1 mM ATPC (P. L. Biochemicals), and 1 U $T_4$ polynucleotide kinase (New England Biolabs). Incubation is at 37° for 60 minutes.

For use as a hybridization probe, maximum specific activity is achieved by substituting 50 µCi, of $\gamma$-$^{32}P$ dATP for the cold ATP as the source of phosphate. In this fashion, the 15-mer can be labelled to specific activity of $1 \times 10^8$ cpm per µg.

10. Dideoxy Sequencing of Plasmid Minipreps

Minipreps of plasmid DNA are prepared according to the method Holmes and Quigley (see Holmes, D. S. et al., *Analyl. Biochem.*, vol. 114,

p. 193 (1981)). Approximately 50 ng of template DNA are digested with PstI. The enzyme is heat inactivated at 65°/10' and 1 µg DNase-free RNase (Sigma) is added. The RNA is degraded for 5' at room temperature at which time the proteins are extracted and the DNA precipitated and dried as described above. Dideoxy sequencing is carried out as follows. Pentadecamer #1 is used as primer in a 20:1 molar excess over template. The primer is annealed to the template in a 13 µl reaction containing 6 mM Tris base pH 7.5; 6 mM $MgCl_2$; 1 mM DTT, and 50 mM NaCl by boiling for 3' and immediately quenching on ice. 10 µCi of $\alpha$-$^{32}P$-dCTP and 1 unit of DNA polymerase Klenow fragment (Boehringer-Mannheim) is added and this reaction is designated the annealing mix. Neutralized 0.5 mM stocks of dTTP, dATP and dGTP (P. L. Biochemicals) are diluted as described below to prepare dN° mixes just prior to use:

| | C° | T° | A° | G° |
|---|---|---|---|---|
| dTTP | 67 µm | 3.3 µm | 67 µm | 67 µm |
| dATP | 67 µm | 67 µm | 3.3 µm | 67 µm |
| dGTP | 67 µm | 67 µm | 67 µm | 3.3 µm |

in 6 mM Tris base pH 7.5; 6 mM $MgCl_2$; 1 mM DTT; and 50 mM NaCl.

Dideoxynucleotide triphosphate (ddNTP-P. L. Biochemicals) mixes are prepared from 5 mM ddNTP stocks just prior to use by diluting with sterile, distilled $H_2O$ to the following final concentration; ddCTP-50 µM; ddTTP-500 µM; ddATP-250 µM and ddGTP-150 µM. The sequencing reaction is assembled from these 3 components, the annealing mix, dN° mixes and the ddNTP mixes, in each of four tubes, labelled G, A, T and C respectively as follows:

| | G | A | T | C |
|---|---|---|---|---|
| dG° | 1 µl | | | |
| dA° | | 1 µl | | |
| dT° | | | 1 µl | |
| dC° | | | | 1 µl |
| ddG | 1 µl | | | |
| ddA | | 1 µl | | |
| ddT | | | 1 µl | |
| ddC | | | | 1 µl |
| annealing mix | 3 µl | 3 µl | 3 µl | 3 µl |

The reactions are incubated at 37° for 15' at which point 1 µl of a solution of 2.5 mM each of dGTP, dATP, dTTP and dCTP (156 µM final concentration) is added. The cold chase reaction proceeds at 37° for 15' and is terminated by the addition of 10 µl of formamide-dye solution consisting of 95% formamide, 0.05% each of xylene cyanol and bromophenol blue, and 10 mM EDTA. The reactions are prepared for electrophoresing by heating to 90° for 2', and quenching on ice. 2-3 µl per lane is loaded on an 8% sequencing gel prepared and ran as follows :

Denaturing polyacrylamide gels (Sanger and Coulson, 1978) are cast in glass plates for use in Bethesda Research Laboratories sequence apparatus. The gel size is 34 cm x 40 cm. The shorter of the glass plates is siliconized with a 5% dichlorodimethylsilane solution in carbon tetrachloride. The gels are 0.4 mm thick and run at constant power (50 watts). Gels are prerun at 50 watts until glass surface temperature reaches approximately 40° C (0.5 hour). Samples are loaded after prerunning using shark tooth combs and 1-2 µl loading volumes. To acquire maximum sequence information from each reaction three gels are run, one 8% and two 6% gels. Eight percent gels are run until the bromphenol blue dye migrates off the gel. A "short" 6% gel is run until the xylene cyanol dye migrates off the gel and the "long" 6% gel is run 5 hours at 50 watts constant power.

After the electrophoresis is complete and power turned off the plates are removed from the apparatus. Tape used to clamp the plates together is removed and the two plates are carefully separated with a

spatula. The siliconized glass plate releases easily from the gel leaving the gel attached to the second glass plate. The gel is recovered from the second glass plate by pressing a dry, developed piece of X-ray film onto the gel and lifting the gel off the glass plate by starting slowly with one corner and peeling the gel away. The gel preferentially adheres to the dry film. A piece of polyester monofilament screen (Tetko, Inc., Rolling Meadows, Illinois) is carefully layed over the gel attached to the film and the screen stapled in place along the sides of the film. At this stage, the sandwiched gel is protected from shifting and can be handled easily. The gel is fixed in a 10% acetic acid/10% methanol bath for 10-15 minutes and washed with water after fixation for an equivalent amount of time. After washing the gel, the screen is removed and the gel recovered by pressing two layers of dry Whatman 3MM paper onto the gel. The gel preferentially adheres to the dry paper and lifts off the wet X-ray film. The gel is covered with saran wrap and dried in a Hoeffer (Model SE1150) slab gel dryer.

Dried gels are exposed at room temperature for 2-4 days and films (Kodak XAR-5) are developed as per manufacturers' recommendations.

B. Results

The primary goal in synthesizing cDNA from mRNA is the generation of full length transcripts, i.e., transcripts which represent the entire coding sequence of the desired gene. Because of inherent differences in mRNA's from different sources, as well as variations from lot to lot in the enzymes used, it will be readily apparent to the ordinarily skilled artisan that various parameters in the following description of each step in the synthesis may require empirical optimization.

<u>First Strand Optimization: KCl Concentration</u>

Effects of KCl concentration on both quantity and length of cDNA transcripts rendered by reverse transcriptase (RT) during first strand synthesis is determined for mRNA isolated from pseudorabies virus (PRV) infected cells and from Bowes melanoma cells. Effect of KCl concentration on quantity and length of transcript are determined in the following manner.

Aliquots from the PRV reverse transcriptase reactions are electrophoresed on a 1.2% alkaline-agarose denaturing gel. Agarose is melted in 50 mM NaCl, 1 mM EDTA and the gel is cast. The solidified gel is

allowed to soak in alkaline electrophoresis buffer (30 mM NaOH, 1 mm EDTA) for 30 minutes prior to use. Samples are dissolved in 50 mM NaOH, 1mM EDTA 12.5% Ficoll and 0.025% bromophenol blue, and electrophoresed.

PRV mRNA clearly favored lower KCl concentrations, yielding maximum mass conversion at 50 mM. KC1 concentrations above 50mM markedly reduced net cDNA synthesis by reverse transcriptase. Likewise, mRNA isolated from Bowes melanoma yields maximum mass conversion at 50 mM, displaying somewhat less sensitivity overall to final KC1 concentration.

Because efficiency of transcription as measured by the incorporation of radiolabelled nucleotide yields no information concerning transcript length, aliquots from the PRV-reverse transcriptase reactions are electrophoresed on 1.2% agarose-NaOH denaturing gels for sizing. A small but clearly discernible increase in high molecular weight material appears as the optional KC1 concentration is approached.

Thus, in terms of both quantity and length of cDNA transcripts, KC1 at 50 mM constitutes the optimal monovalent cation concentration for these mRNA's. Other mRNA's may have different requirements. Retzel et al., Bio. Chem., vol. 19, pp. 513-8 (1980), have demonstrated the ability of reverse transcriptase to synthesize transcripts as long as 7200 nucleotides from AMV (avian myeloblastosis virus) mRNA in the presence of 148 mM KC1. Because reverse transcriptase is capable of functioning over a wide range of monovalent cation concentrations, and seems to display different requirements for mRNAs from different sources, empirical optimization of this parameter is recommended.

Enzyme Concentration

Effects of increasing amounts of reverse transcriptase on rate and extent of first strand synthesis are determined. Two reactions containing moderate levels of enzyme and one containing a substantial excess of enzyme are assayed for rates of incorporation and total cDNA made.

As expected, increasing the amount of enzyme increased both rate and extent of cDNA synthesis. Unexpected, however, are the percent mass conversion data. Minor errors in determining the amount of mRNA going into these reactions is inadequate to explain a 280% mass conversion. The possibility that second strand synthesis is occurring during

the first strand reaction is tested by treating these RT products with NaOH to degrade their mRNA templates and testing their sensitivity to the single strand specific endonuclease $S_1$. All three reactions demonstrate the production of some $S_1$ resistant material. Most striking, however, is the observation that in the presence of vast enzyme excess 56% of the counts incorporated by reverse transcriptase are $S_1$ resistant. Clearly at high enzyme concentrations reverse transcriptase is capable of displacing the mRNA template and synthesizing the second strand. While not tested, the possibility exists that in the presence of the negligible RN'ase H activity associated with the RT used, the displaced mRNA template again becomes available for additional "re-initiation" transcription. This would explain mass conversions above the theoretical double strand limit of 200%.

Reverse transcriptase products, whether complete or partial copies of the mRNA template, often possess short, partially double stranded hairpins at their 3' termini (see Wickens, M. P. et al., J. of Biol. Chem., vol. 253, pp. 2483-95). These hairpins are required as primers by DNA polymerase Klenow fragment for the extension of the second strand (see Efstradiatis, A. et al., Cell, vol. 7, pp. 279-88 (1976) and Rougeon, F. et al., Proc. Nat. Acad. Sci., vol. 73, pp. 3418-22 (1976)). In order for them to form and become available to the Klenow enzyme, the mRNA template must be removed prior to attempting second strand synthesis with this enzyme.

Template Removal

The method of this invention for removing the mRNA template prior to second strand synthesis uses globin mRNA (Bethesda Research Labs) as a substrate. The first strand is synthesized as described above. Subsequently, NaOH is added to 0.3 M and is incubated at 42° for 60 minutes. mRNA template degradation is a function of the efficiency with which the first strand mediates second strand synthesis. From a comparison of globin cDNA on a standard, nondenaturing 5% acrylamide gel shows mRNA template removal by the complete shift-down of material from the aberrantly migrating single stranded transcript to the 700 basepair double stranded globin cDNA. The result is a single strand cDNA capable of functioning as a template directing second strand synthesis as indicated by identical incorporation of $^3H$-dCTP (1.3%)

during the Klenow reaction. Control reactions attempting to synthesize second strand without prior removal of the mRNA template shows little or no 3H-dCTP incorporation.

Second Strand Synthesis

Second strand synthesis mediated by DNA polymerase Klenow fragment is monitored routinely by incorporation of $^3H$-dCTP. It is repeatedly found that at 15° the reaction goes to completion by 4 hours and additional reaction time usually does not effect the result and could, depending on the particular preparation of Klenow, result in a loss of as much as 30% of TCA precipitable material.

In keeping with the desire to obtain double stranded transcripts of as great a length as possible, material from the Klenow reaction is subjected to an additional hour of polymerization at 50° C in the presence of reverse transcriptase. It is hoped that secondary structure sufficient to stop Klenow at 15° C would be melted out at 50° C thus allowing reverse transcriptase to complete any unfinished second strands. The reaction is monitored by measuring additional 3H-dCTP uptake over the course of the 60 minute reaction. It is consistently found that for globin or Bowes mRNA, no additional incorporation occurs. While no measurements are made with PRV mRNA, it is reasonable to expect that the addition of a high temperature second strand RT step would be of benefit in unravelling the unusually tenacious secondary structure associated with high G-C mRNAs. Hence the additional RT step is routinely added when working with such templates.

S1 Endonuclease

In order to generate a double stranded cDNA molecular suitable for cloning (see "tailing" below), the hairpin used for priming second strand synthesis must be eliminated. This is accomplished using the single strand specific endonuclease $S_1$. Because this enzyme is reported to be sensitive to concentrations of deoxynucleoside triphosphates (dNTP's) as low as 1 μM (see Weigand, R. C. et al., J. Biol. Chem., vol. 250, pp. 8848-55 (1975)), unincorporated dNTPs left over from the polymerization reactions must be efficiently eliminated. Due to variability in recovery efficiencies, as well as the inconvenience associated with many types of sizing columns, an alternative means of disposing of dNTPs is used. The most straightforward means of

accomplishing this is to take advantage of the greater solubility of dNTPs vs. cDNA in the presence of 2.5M $NH_4Ac$, 70% ethanol. cDNA purified on Sephadex G-50-medium, and cDNA purified by successive $NH_4Ac$, NaCl-ethanol precipitations are used as substrates in $S_1$ reactions involving increasing amounts of $S_1$. Reactions are setup as described in "Materials and Methods" under "t-PA cDNA" above, each containing 6 ng of double stranded globin cDNA and $S_1$. Aliquots from the reactions on 1.2% alkaline agarose gels next to single stranded globin cDNA. Electrophoreses is accomplished here in a manner to that used in determining the effect of KCl concentration above. No consequential difference in $S_1$ sensitivity is observed over the range of $S_1$ concentrations used, indicating that ethanol precipitation performed as described, is just as effective at removing unincorporated nucleotide as the Sephadex G-50 column for purpose of $S_1$ digestion.

An experimental format similar to that described above is used to titer each lot of $S_1$ nuclease received prior to use in actual cDNA constructions. Briefly, $S_1$ in increasing amounts is reacted with known, constant mass amounts of double stranded cDNA and the reaction products are electrophoresed on alkaline-agarose gels along side single stranded cDNA. The proper ratio of S1: cDNA is defined as the minimum amount of enzyme required to cause the double stranded cDNA to co-migrate with the single stranded cDNA on denaturing gels (an observation which would be consistent with clipping the hairpin, thereby reducing its denatured size to equality with that of its single stranded percursor). It is clear that a decrease in material running at "2x" and a proportional increase in material running at "1x", results from increasing amounts of $S_1$. Ordinary experimentation easily shows what is considered to contain the proper amount of enzyme for efficient removal of the hairpin. Because $S_1$ endonuclease preparations usually have varying amounts of double strand exonuclease either integrally associated with the enzyme or co-purifying with it, titration of each lot of $S_1$ should be routine. The consequences of over digestion with $S_1$ are apparent in the following material.

Sucrose Gradients

Double stranded $S_1$-treated cDNA is routinely sized on sucrose gradients. This allows additional enrichment for cDNAs of a desired

size class as well as eliminating very small material which interferes with subsequent steps.

Terminal Transferase

The addition of homopolymer tracts of dCTP residues ("tails") of appropriate length (10-30 residues per end) using deoxynucleotidyl transferase (dTT) is perhaps the most difficult in the entire series of cDNA reactions. The basis for this lies in the very limited range over which tail length can vary while still permitting the cDNA to represent an optimal substrate for cloning in E. coli. Factors further complicating control of the reaction are: (1) the high rate of incorporation of dCTP; 17 pmoles per minute per unit of enzyme (substrates not limiting), (2) the sensitivity of the enzyme to dilution, (3) the resultant need to reduce reaction rate by severely limiting substrate (dCTP) concentration as well as reducing the temperature of the reaction, (4) the fact that the calculation of residues added per 3'-end relies very heavily on crude approximations of mass amount and average size of cDNA in the reaction, and finally (5) the fact that these calculations make the assumption that all 3'-ends are tailed with equal efficiency - an assumption which is most likely inaccurate.

The best way to titer the terminal transferase reaction is by using actual, heterogenous cDNA material. For the sake of the necessary calculation of residues added/3' terminus, assumptions are made as described above. In essence, once approximate parameters are empirically determined, each tailing reaction is approached conservatively, cycling through incubations and tail length determinations until that particular cDNA is appropriately tailed.

Two technical notes should be added at this point. First, dTT continues to add residues even at 0° C. Since the enzyme is capable of withstanding repeated freeze thaws without noticeable loss of activity, it is recommended that while determining tail length the reaction be stopped by freezing immediately on dry ice. Secondly, cacodylic acid (arsenic) is toxic to E. coli. Ethanol precipitation, preferably prior to annealing, (carrier tRNA may be added at this point) is absolutely essential.

t-PA cDNA

Initial cDNA banks (primed with oligo $dT_{12-18}$) are generated from

mRNA enriched for t-PA sequences by fraction on sucrose gradients and subsequent assay of oocyte translation products via the casein-agar plate method as described by Miskin, R. et al., Nuc. Acids Res., vol. 9, 3355-63 (1981). t-PA activity is rendered by mRNA migrating at about 19S (see Opdenatker, G. et al., Eur. J. Biochem., vol. 121, pp. 269-274 (1982)) and of 300 µg of Bowes polyA + mRNA loaded onto the gradient, about 50 µg are pooled from peak fractions. Ten µg of this enriched mRNA yields approximately 3 µg of cDNA. After size fractionation of this cDNA, about 100 ng (average size of 1000 bp) are available for cloning. Transformation efficiency with this cDNA is somewhat low at 6x103 transformants/µg cDNA, probably as a result of suboptimal tailing conditions being used at the time. Two additional banks are similarly constructed resulting in a total of 8000 transformants, with a background of about 4%. (Percent background is the quotient of the number of transformants obtained via transformation with self-annealed vector divided by the total number of transformants.) 20,000 colonies derived from the three banks are screening using a 32p-labelled pentadecamer complimentary to the coding sequence of the last 5 amino acid residues of the protein (nucleotides 1759-1773). Of these, one colony hybridized is duplicate. Pstl restriction analysis of this clone is performed to determine if the digestion products are consistent with the Pstl-restriction map (Chart 6). The digest yielded 4 fragments: the original pBR322 vector, an -1150 bp fragment, an -80 bp fragment and a -78 bp fragment. These results suggest an insert spanning -1300 bp of the gene's 3'-end (nucleotides 1250-2550). To confirm this assumption, the clone is double digested with Pstl and Ddel. The latter enzyme should cut the 1150 bp fragment into a 926 bp fragment and a 229 bp fragment while leaving the 78 or 80 bp fragments intact. The results of such a double digest support the conclusion that the clone does in fact represent the 3'-end of the t-PA gene.

As final proof, a mini-preparation of DNA is isolated from the clone and is sequenced by the dideoxy chain termination protocol as described above in "Materials and Methods" under "t-PA cDNA." The sequence data derived from this clone, designated pTPA H, are identical to sequence data presented by Pennica et al., Nature, vol. 301, pp. 214-21 (1983) for human tissue plasminogen activator.

From here the focus is on isolation of the 5'-end of the gene. To do this, cDNA is synthesized from twice-selected polyA + mRNA by primer extending from pentadecamer #1 rather than from $OdT_{12-18}$. The advantage afforded by this approach is twofold. First, because priming is specific for t-PA, a higher percentage of the cDNA made should be represented by t-PA specific sequences. Secondly, priming is some 800 bp 5' of the polyA tail, the site utilized by $OdT_{12-18}$ to prime first strand synthesis. This almost assures that the cDNA derived from this approach will include transcripts extending further 5' than pTPA H.

To test the specificity of priming, reverse transcriptase reactions are set up containing either no primer, $OdT_{12-18}$ as primer, or t-PA pentadecamer as primer. Incorporation of $_{32}P$-dCTP into TCA precipitable material is monitored. The data indicate that with Bowes mRNA, a fair amount of self priming occurs, even after two selections on Odt-cellulose.

Substracting this from the incorporations obtained in the presence of each of the 2 primers, about half as much cDNA is made when specifically primed with t-PA primer as with $OdT_{12-18}$. Considering that t-PA specific sequences are thought to represent a very small percentage of polyA + mRNA (~0.01%), this observation suggests that self-priming may be enhanced by the primer:template annealing procedure used in this reaction (see Materials & Methods) of t-PA cDNA above. Aliquots of these 3 RT products as well as from a separate experiment generated by priming at a different point in the t-PA gene are electrophoresed on denaturing gels. The appearance of discrete bands (in addition to the broad "smear" indicative of the normal size heterogeneity of a transcript population) would be suggested of both specificity of priming as well as enrichment for the primed transcript.

The first gel compares single stranded cDNA synthesized in the 3 reactions described above. Discrete bands appearing at about 1750 and 2100 nucleotides do in fact appear in the cDNA primed with the t-PA 15-mer (15-mer #1) (a full length transcript synthesized from this primer would be about 1775 nucleotides in length). These bands are absent in the cDNA nonspecifically primed with $OdT_{12-18}$, suggesting both specificity of priming by the 15-mer, and therefore, enrichment of t-PA sequences during transcription. The bands are also absent from a self-primed cDNA population. Likewise, discrete bands of ~200, 350, and ~450

nucleotides in length appear in cDNA primed with a pentadecamer complimentary to a different area of the t-PA gene (15-mer #2). Together these data strongly support the contention that the discrete bands represent an enrichment of t-PA-specific transcripts, and are not artefacts arising from non-specific or self-priming of the mRNA.

Transformation efficiency with this t-PA-primed, cDNA is 2.5x10$^5$ transformants per µg of cDNA. Two banks totaling 25,000 transformants are generated. The concept of "gene-walking" is applied to purposely bias the screening. The pTPA H 5'-terminal 80 bp Pstl fragment is gel isolated and used to probe 28,000 colonies derived from the primer extended banks. This specifically selects for clones extending at least as far 5' as pTPA H, and hopefully as a result of the primer extension approach, clones containing additional 5' sequences as well.

The screen yielded 50 positives. This represents a 16 fold increase in t-PA specific clones when compared with $OdT_{12-18}$ primed cDNA synthesized from t-PA enriched (via oocyte injection) mRNA. The longest t-PA insert is found during secondary screening with Pstl and Ddel. The clone, designated pTPA80-1, included an insert spanning nucleotides 550-1600. The data thus clearly indicates pTPA80-1 which spans the t-PA sequence from nucleotides 550-1600, which is consistent with Chart 6 depiction.

The restriction data make it possible to determine fairly accurately (+/- -10%) the position of the 3' terminus. If priming begins at nucleotide 1759, and pTPA 80-1 has a 3' terminus at about position 1600, then a loss of approximately 160 basepairs occurres at some point during the cloning. The $S_1$ endonuclease is the most likely cause of the loss. It's not clear from these experiments, however, that the loss occurres due to infidelity of the single stranded specificity of the enzyme. It's certainly possible that for some reason second strand synthesis stopped at nucleotide 1600, meaning that the single stranded DNA template extending 3' from position 1600 to the original priming site would be a legitimate substrate for $S_1$. The more likely explanation in light of the compound second strand synthesis reactions used in making this cDNA (Klenow followed by high temperature RT) is that overdigestion with $S_1$ results in enhanced double stranded nuclease activity. If this in fact is the case, it might be assumed that degradation proceedes from both ends, meaning that the double stranded

cDNA transcript is reduced in length by ~320 bp. In either event, it's apparent that cDNA synthesis without the requisite $S_1$ step would result in transcripts of greater length. Alternatives are discussed below.

The orientation of pTPA H and pTPA 80-1 are determined and are depicted in Chart 7 along with the strategy devised to combine them. The new construct, designated pTPA, is verified by Pstl digestion.

Since pTPA 80-1 ~~is the~~ longest t-PA insert amongst the 50 positives detected in the first primer extended banks, a second oligonucleotide complimentary to the coding sequence of amino acid residues 233-237 (nucleotides 886-900; 15-mer #2) is used to generate another cDNA bank. This particular area of the gene is chosen so that should $S_1$ be removed even as much as 200 bp from the 3' terminus of the transcript, sufficient overlap with pTPA L would still exist so as to allow for assenbly of the fragments.

Transformation efficiency with this cDNA is 3.6z$10^4$ transformants/μg cDNA and a total of 6300 colonies (background of 3%) is obtained. About 7000 colonies from this bank are screened with an oligonucleotide complimentary to coding sequence extending from nucleotides 645-660 (15-mer #3). Again about 50 unambiguous positives are obtained. (This represents a 50-fold enrichment of t-PA-specific transcripts over cDNA nonspecifically primed with $OdT_{12-18}$). Secondary screening is accomplished by Pstl and BglII digestion and 1 clone, pTPA 12, is shown to contain the remaining 5' sequences. Again, this is consistent with the Pstl-restriction map for t-PA shown in Chart 6. Again, the effects of $S_1$ on insert length are apparent with pTPA 12. While priming is initiated at nucleotide 886, pTPA 12 extends only to about nuicleotide 750; a loss of about 136 bp.

A linear summary of all t-PA inserts derived above is illustrated in Chart 8.

With the entire t-PA sequence available on two clones (pTPA L and pTPA 12) the final task of assembly remained. The strategy is depicted in the following outline:

1. 5' End pTPA 12

A.

```
                Taq I
pTPA 12 ---------→ GEL PURIFY 2194 bp fragment
               (20 μg)
```

B.

```
                BglII
2194 ------------→ GEL PURIFY 405 bp fragment
                HgaI
```

II. CENTER SEQUENCE; pTPA L

A.

```
                         PvuII
pTPPA L -----------------→ GEL PURIFY 1748 bp fragment
              (100 µg) EcoRI
```

B.

```
                HgaI
1748 ------------→ GEL PURIFY 209 bp fragment
```

III. 3' END: pTPA L

```
                 T4 DNA Paymerase   EcoRI PARTIAL DIGEST
pTPAL ---→ Linear 63Kb ---→ FLUSH-ended ---→GEL PURIFY 1871 bp Fragment
     PvuI            4 dNTP's

(20 µg)    3' overhand        linear
```

IV. VECTOR: pKC7

```
pKC7----------→ 4.8 Kb + 1 Kb ----------→ VECTOR

      BglII SmaI                Bacterial Alkaline
                                  Phosphatase
```

Each of the above products I., II., III. and IV. are depicted in Chart 9 and then shown as ligated t-PA fragments in Chart 10. One noteworthy aspect of this strategy is the means by which the need for alkaline phosphatase treatment of the internal fragment is obviated.

Typically, multipiece (≥ 4 fragments) ligations produce the desired construction with very low efficiency due to concatemerization of the fragments. Treatment with alkaline phosphatase in the appropriate fashion minimizes this problem, resulting in a dramatic increase in efficiency of correct, multipiece assemply. (See Clark-Curtis, G. E. et al. cited above). In assembling the t-PA fragments, we took advantage of the unique way in which the restriction endonuclease, Hgal, cuts DNA. The recognition

(binding) sequence of the enzyme is GACGC but the enzyme cuts at a point beyond that recognition sequence (Chart 11). As a result, fragments cut with Hgal will ligate only with their originally contiguous counterpart. Self-ligation promoted by the Hgal overhang cannot happen. The 4-piece t-PA ligation contains two fragments bearing an Hgal terminus. That in addition to treatment of the vector with alkaline phosphatase (to minimize self-closure) results in >80% of the transformants bearing the correctly assembled t-PA gene. Correct assembly is verified by the restriction digests. The plasmid, designated pTPA, is illustrated in Chart 12.

Discussion

The task of eukaryotic gene isolation exercises virtually every technical skill currently associated with state-of-the-art nucleic acid biochemistry. However, the method described herein emphasizes those techniques which provide the means for circumventing constraints imposed on gene isolation strategies both by relative sequence abundance as well as by eukaryotic gene topography (i.e., introns); specifically, the use of oligonucleotides in enriching for and isolating rare transcripts and the in vitro conversion of nRMA isolated from an enriched source into a population of double stranded DNA copies. Thus, the gene source together with the detailed, complete techniques used to isolate the t-PA gene herein which are known and available to the public provide one of skill in the art enabling disclosure. In summary, several factors can greatly facilitate this process. Ready access to tissue which is synthesizing large amounts of t-PA the target proteins is known. Further, proteins synthesized by established cell lines as here in the Bowes melanomas cell line are particularly convenient.

Chart 1: Construction of pTPAH

(XV) pTPA having 7.4 kb

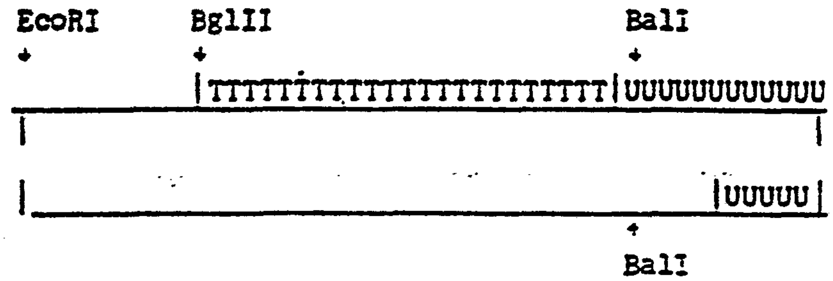


(I) pTPA SalI

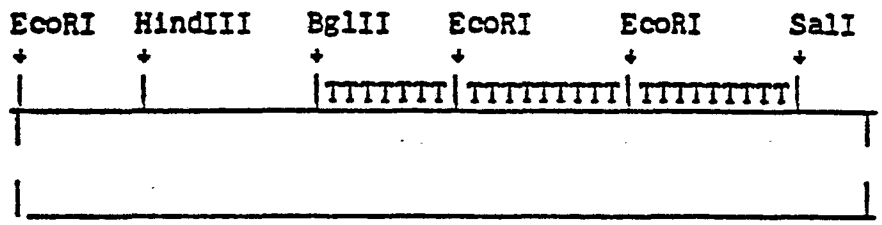


(II) pTPAH

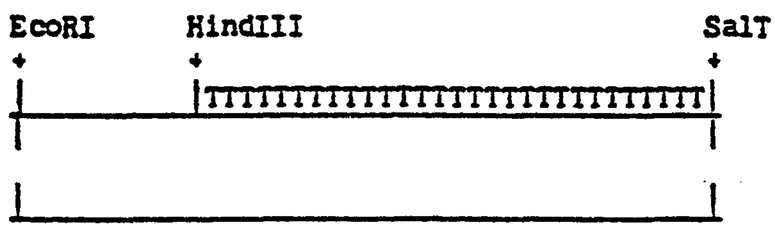


(XV) Cut with BalI and Ligate SalI linkers → (I) Cut with BglII; fill ends partially; Treat with Mung bean nuclease; Cut with HindIII; fill ends and ligate; Transform _E. coli_ and screen → (II)

TTTTTTTTTTTTTTTTTTTTUUUUUUUUUUUU = t-PA gene.

TTTTTTTTTTTTTTTTTTTTT = Coding region of t-PA gene.

UUUUUUUUUUUU = 3'-Untranslated region of t-PA gene.

Chart 2: Plasmid pal-ADHt

(III) pal-ADHt

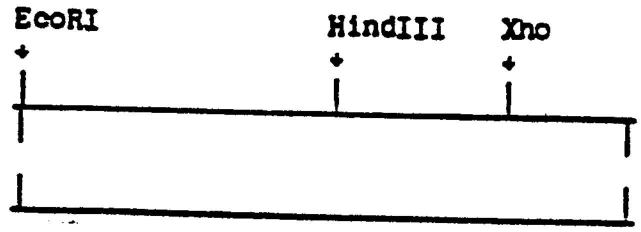

Chart 3: Modification to the t-PA gene

The t-PA gene

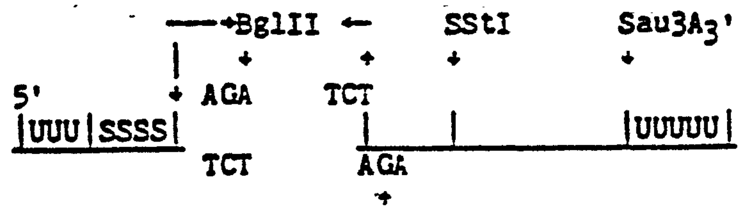


SSSS = Signal sequence.

UUUUUUU = Untranslated region of t-PA gene.

Chart 4: Construction of the Plasmid pYREP31B

(IV) Y1P31 Plasmid

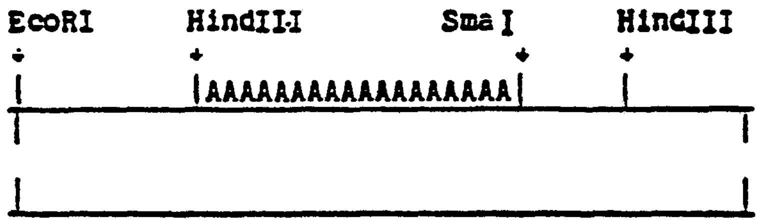


(V) 2μ plasmid from yeast

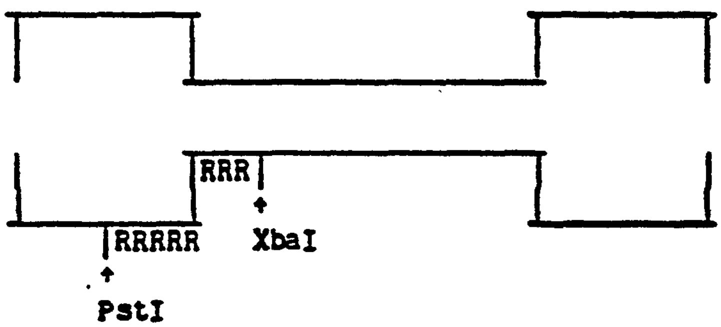


(VI) pYREP31B

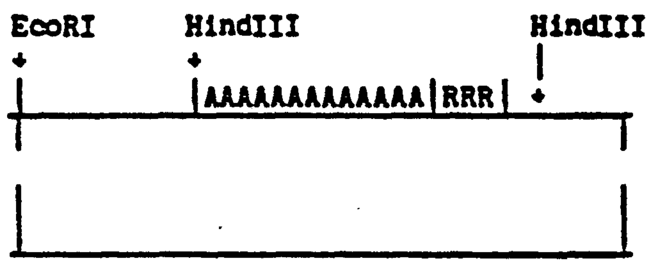


(IV) Cut with SmaI; 'BAP'

(V) Cut with PstI and XbaI purify 1.3 Kb fragment

→ ligate and transform into E. coli → (VI)

AAAAAAAAAAAAA = URA3.

RRRRRRRR = Fragment spanning the REPIII and replication origin.

Chart 5: Construction of Plasmid PGG400

(VII) pBR322

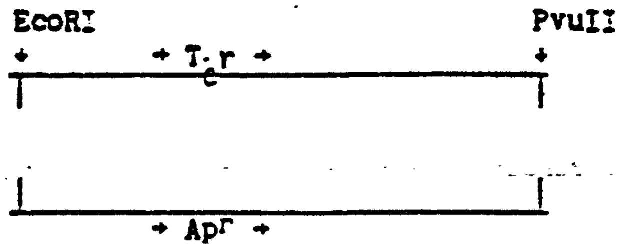


(VIII) pmL21

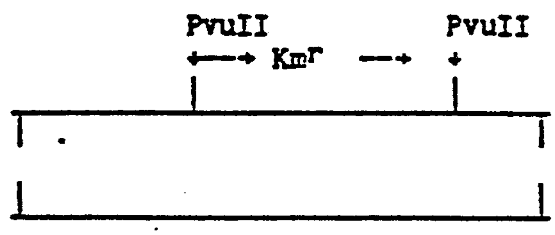


(IX) pGG400

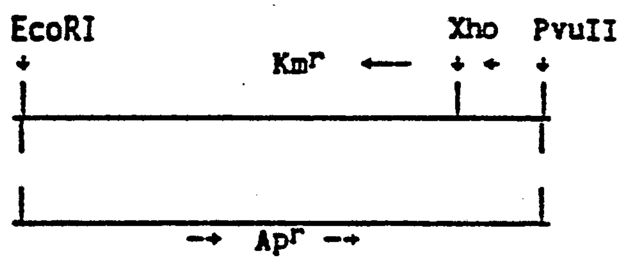


(VII) Cut with EcoRI and PvuII
Fill ends

(VIII) Cut with PvuII

→ ligate; transform into *E. coli*; select for $Km^r$; $Tc^s$ clone → (IX)

$Km^r$ = Kanamycin resistance.

$Ap^r$ = Ampicillin resistance.

$T_c r$ = Tetracycline resistance.

Chart 6: Construction of pADHt

(IX) PGG400 (see Chart 5)

(X) pADHBC

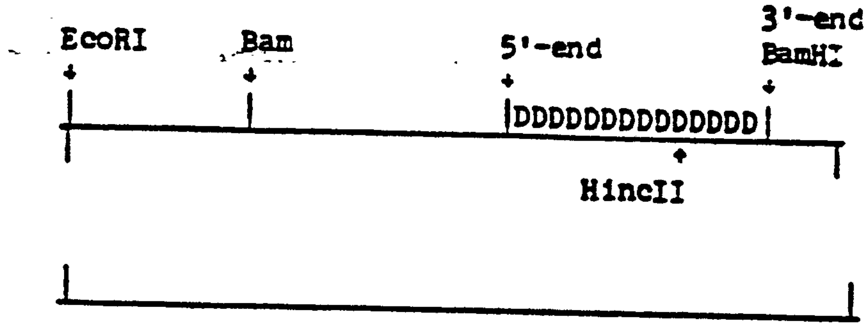


($XI_1$) pADHt

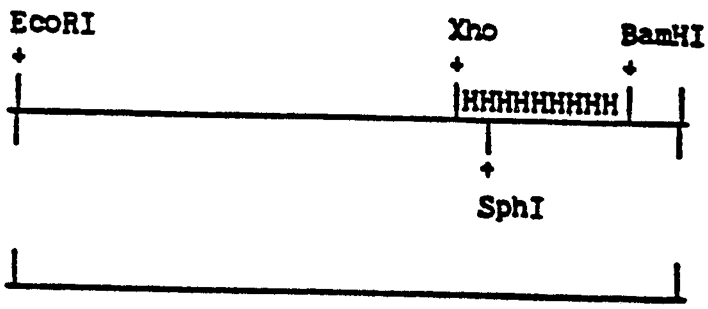


(IX) Cut with Xho and PvuII;
Fill ends

ligate, transform from *E. coli* and screen → (XI)

(X) Cut with HincII and BamHI;
Fill ends with $T_4$ pol.

DDDDDDDDDDDDDDD = ADHI.

HHHHHHHHHHH = ADHI 3'-end.

Chart 7: Construction of Plasmid pADHt-REPIII

(XI$_2$) Modification of Plasmid pADHt (XI$_1$) shown in Chart 6.

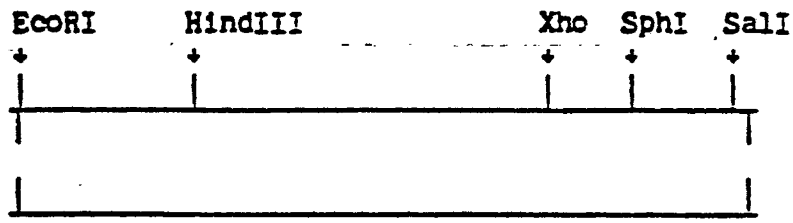


(VI) pYREP31B (see Chart 4)

(XXVII) pADHt-REPIII

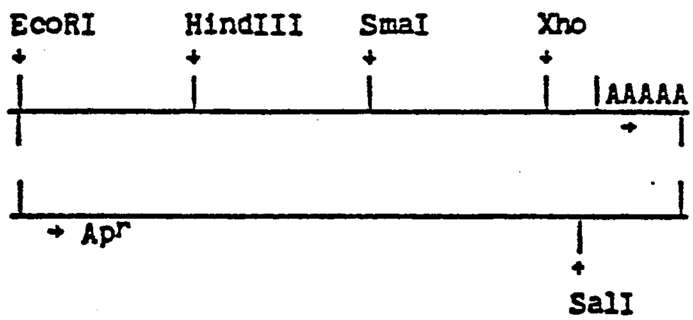


(XI$_2$) Cut with SphI; Fill ends with T$_4$ pol.

ligate and transform into *E. coli* → (XXVII)

(VI) Cut with HindIII Fill ends with T$_4$ pol.

AAAAAAAA = URA3 gene.

Chart 8

(XXVII) pADHt-REPIII (see Chart 7)

(XXVIII) pα1

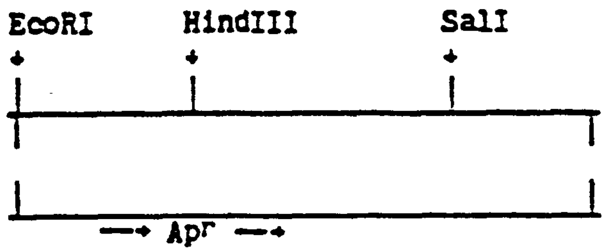


(III) pα1-ADHt approximately 6.8 Kb by size

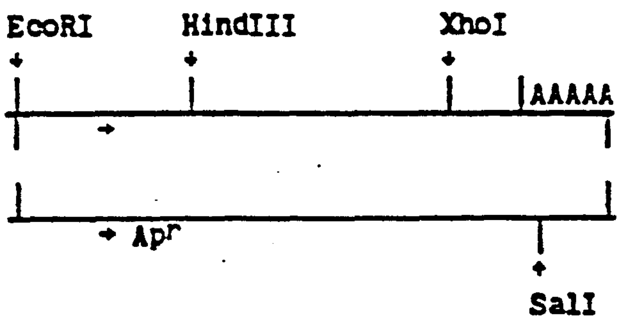


(XXVII) Cut with EcoRI-HindIII
Purify the larger fragment

→ ligate and transform into *E. coli* → (III)

(XVIII) Cut with EcoRI-HindIII
Purify the smaller (1.2 Kb) fragment

AAAAAA = URA3 gene.

Apr = Is ampicillin resistance gene.

Chart 9

(III) pα1-ADHt (see Chart 8)

(II) pTPAH (see Chart I)

(III')

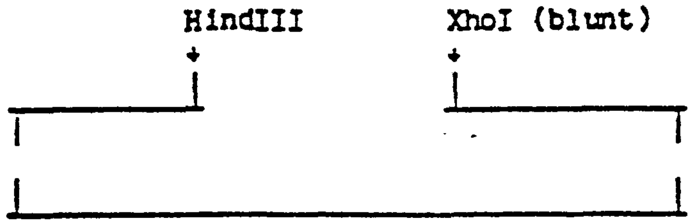


(II')

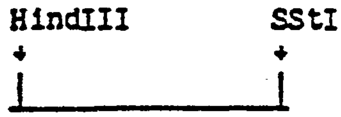


(II'')

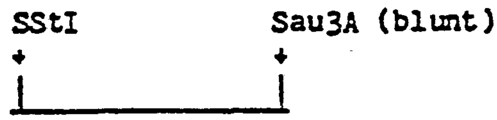


(XXX) pα1ADHt-TPA

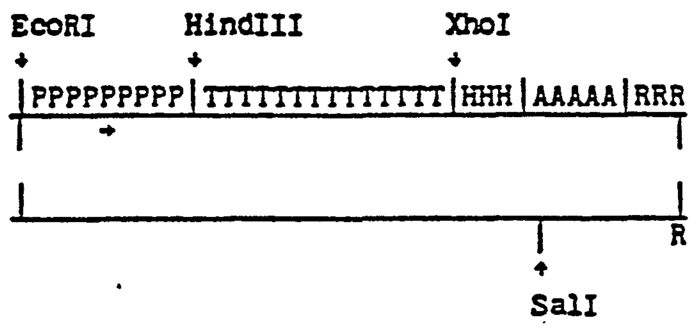


PPPPPPPPPPPPP = MFα1 promoter and "pre-pro" signals.
TTTTTTTTT = DNA encoding for t-PA.
HHHH = ADHI 3'-end.
AAAAAAAA = URA3 gene.
RRR = REPIII and replication origin from 2μ yeast plasmid.

(III) Cut with XhoI and fill ends then cut with HindIII and purify the larger fragment → (III')

(II) Cut with HindIII/SStI purify the smaller fragment→(II').

(II) Cut with Sau3A/SStI as described and purify 389 bp Sau3A/SStI fragment → (II'').

(III')
+
(II')
+
(II'')

→ ligate, transform *E. coli* HB101 with ligated DNA and screen for appropriate plasmid → (XXX).

Chart 10

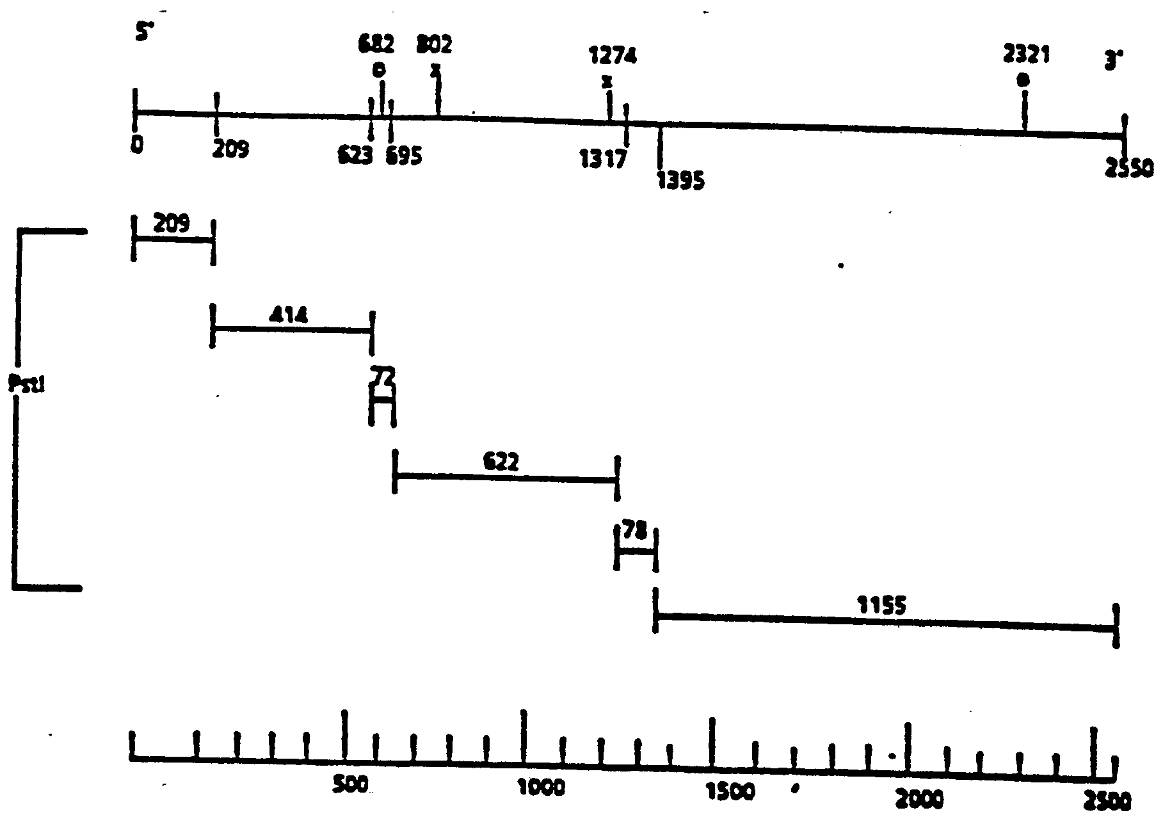


PstI - Restriction MAP: t-PA
= DdeI (all sites not shown)
= EcoRI

Chart 11: Orientation and Assembly of TPA H and pTPA 80-1

(XII) pTPAH having 5.7 Kb

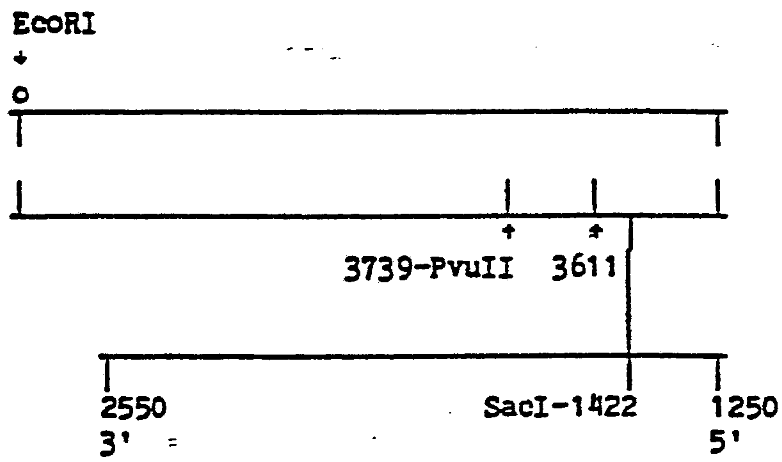


(XIII) pTPH 80-1 having 5.4 Kb

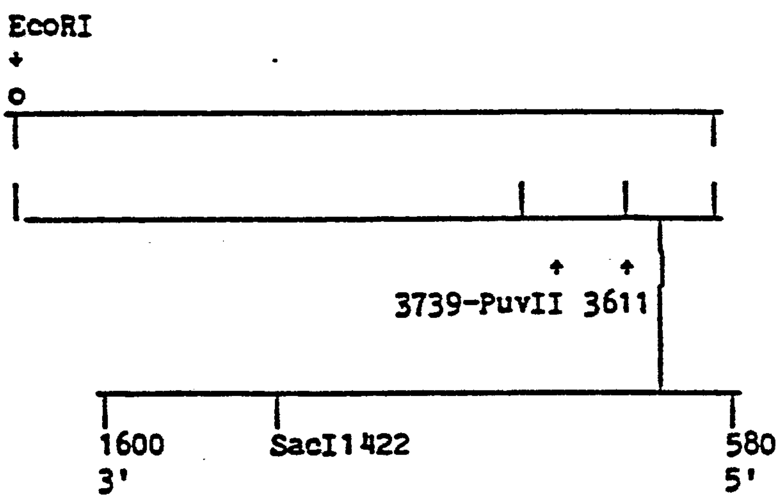


(XIV) pTPAL having 6.3 Kb

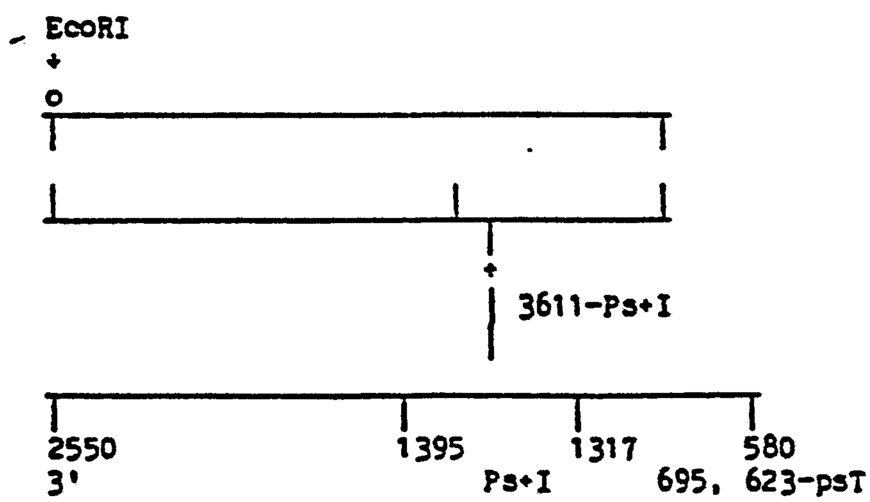

5'
Chart 12 Insert summary of 4 partial t-PA clones

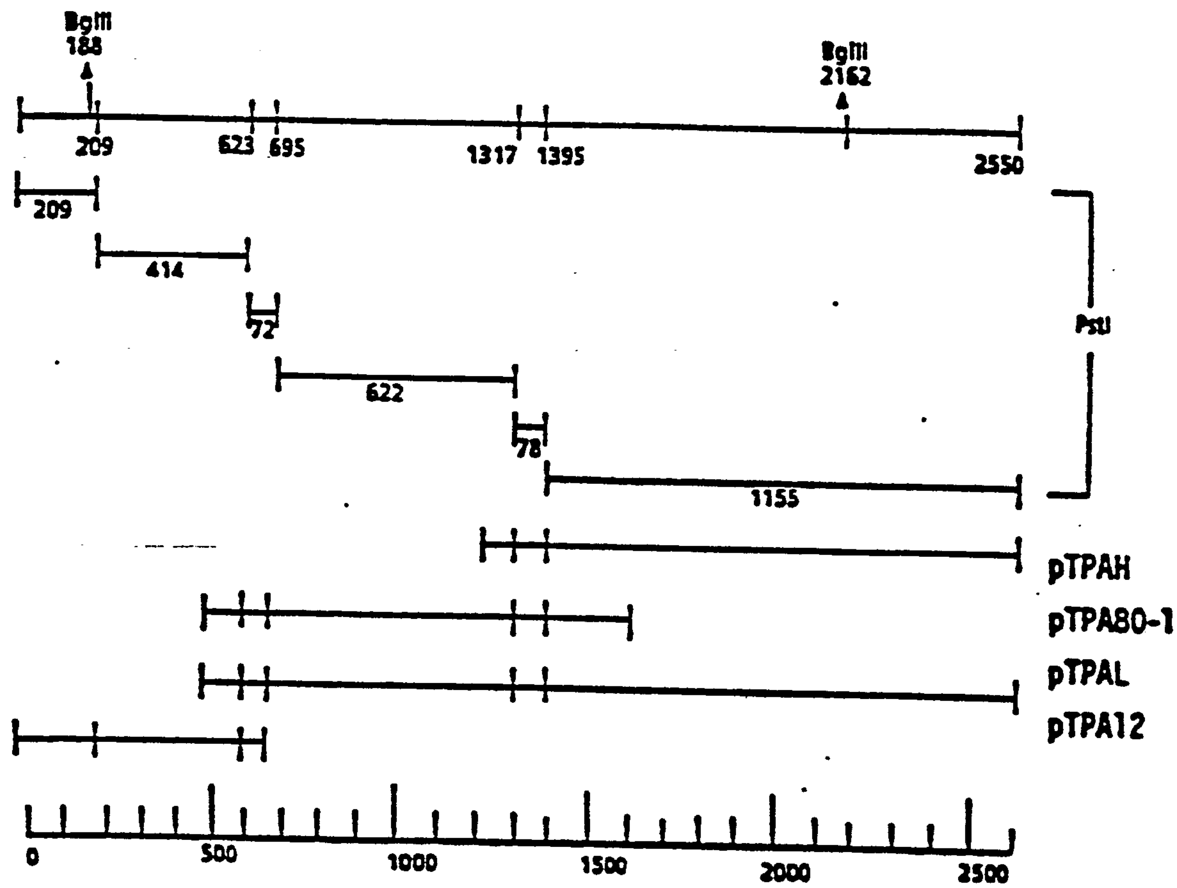

Chart 13

(XV)

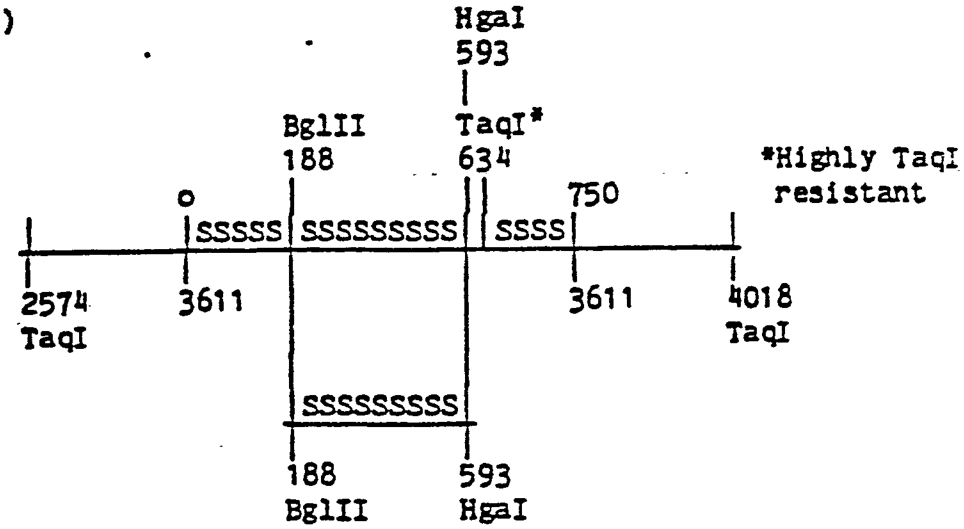


(XVI)

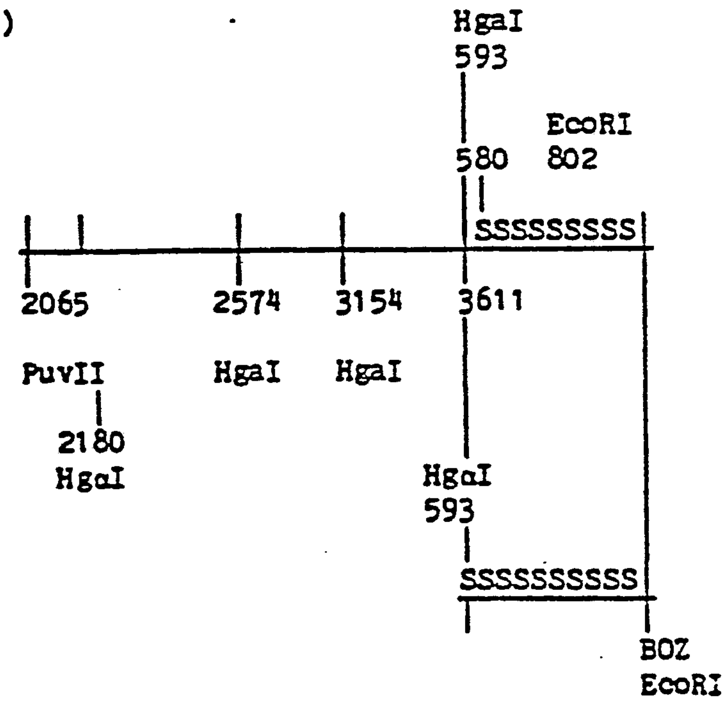


(XVII)

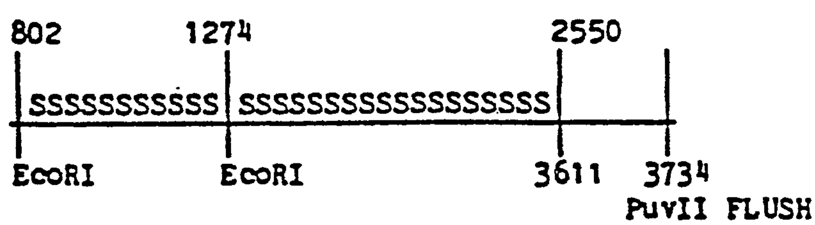


(XVIII)

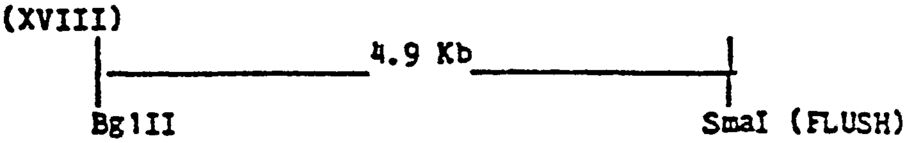


SSSSSSSSS = Solid mark merely to highlight a particular plasmid fragment.

Chart 14: Ligation of t-PA fragments

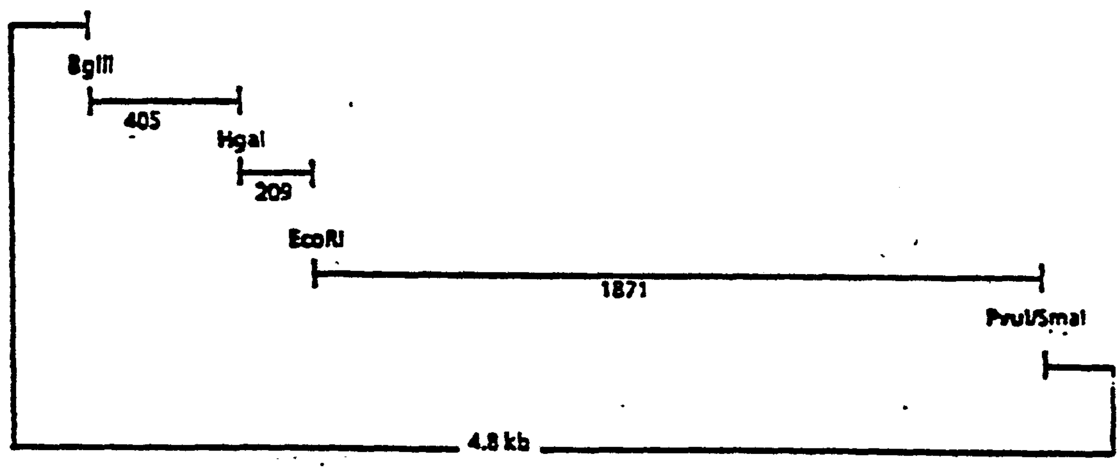

Chart 15: Recognition and cutting sequence of the restriction endonuclease HgaI. Arrows indicate sites of strand scission

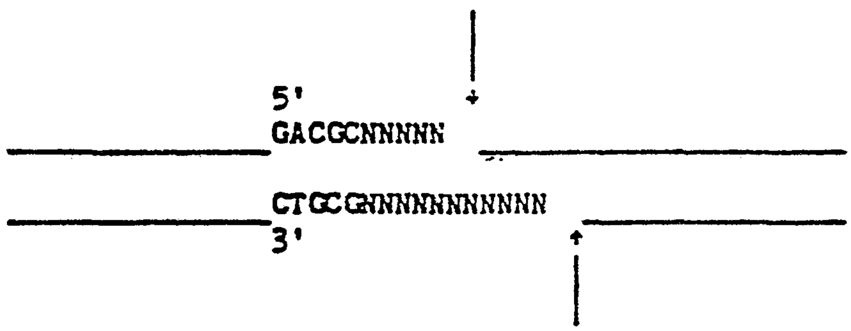

## Chart 16: Map of pTPA

(XV) pTPA having 7.4 Kb

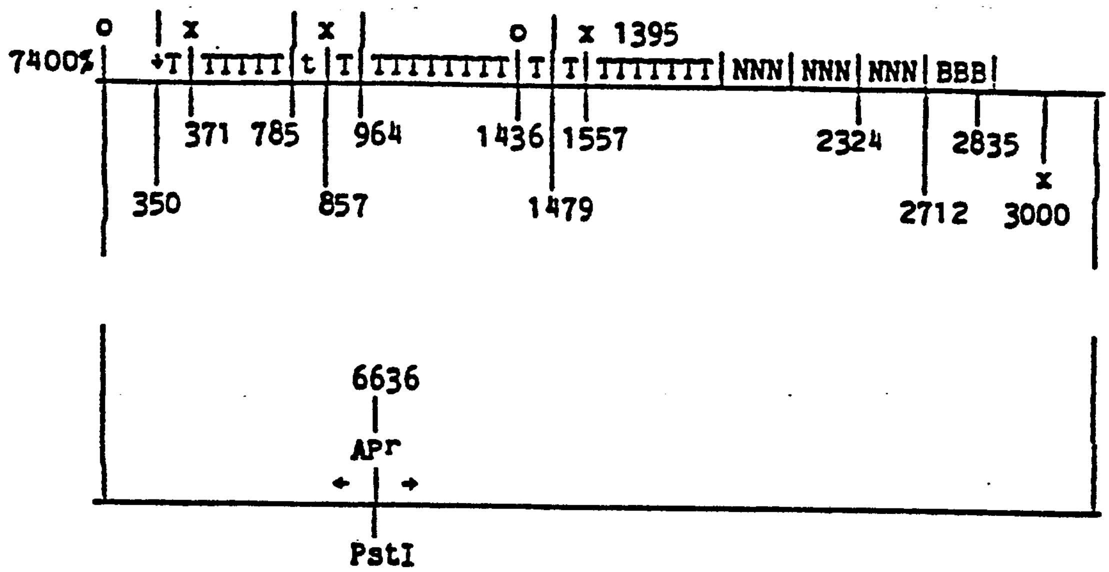


o| = EcoRI.

x| = PstI.

BBBB = pBR322.

TTTTTTTT = t-PA coding sequence.

NNNN = t-PA 3' non-coding sequence.

CLAIMS

1. t-PA having selected glycosylation.

2. t-PA when expressed by yeast.

3. A t-PA according to Claim 2 wherein the yeast is Saccharomyces cerevisiae.

4. A circular recombinant DNA compound of formula XXX

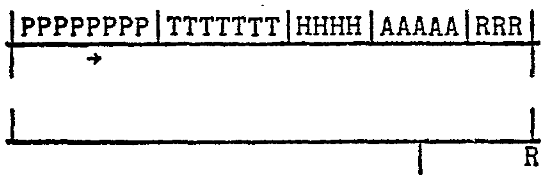


(XXX)

wherein the repeated alphabetic sequences (the number of repetitions of which is arbitrarily depicted in said formula) therein represent the following:

the "P" region comprises nucleotides whose sequence contains a yeast promoter and "pre-pro" signals consisting essentially of the "pre-pro" signals from the MFα1 gene;

the "T" region comprises nucleotides whose sequence contains DNA for the synthesis of t-PA;

the "H" region comprises the nucleotide whose sequence contains the 3'-end of the ADHI gene;

the "A" region comprises the nucleotides whose ordered codons contain the URA3 gene;

the "R" region comprises nucleotides whose sequence contains a REPIII and replication origin from the 2μ yeast plasmid.

5. A compound of Claim 4 wherein the "T" region does not contain the 3'-end untranslated region of the DNA for the synthesis of t-PA.

6. A yeast cell or cell line whose expressed genetic material comprises a DNA compound of Claim 4.

7. A cell or cell line according to Claim 8 wherein the DNA compound of Claim 4 expresses t-PA.

8. A method of producting t-PA which comprises culturing the cell line according to Claim 7.

European Patent Office

# EUROPEAN SEARCH REPORT

0174835

Application number

EP 85306432.7

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X,D | EP - A1 - 0 093 619 (GENENTECH, INC.)<br>* Claims 1-4,6 *<br>-- | 1,2,4, 7,8 | C 12 N 9/48<br>C 12 N 9/60<br>C 12 N 15/00<br>C 12 N 1/16<br>C 12 N 5/00<br>//C 12 R 1:865 |
| X | EP - A2 - 0 037 687 (ABBOTT LABORATORIES)<br>* Claims 1-3,8-10,22-24 *<br>-- | 1-3,6-8 | |
| A | EP - A2 - 0 117 059 (GENENTECH, INC.)<br>* Abstract; claim 1 *<br>-- | 1-4 | |
| A,D | NUCLEIC ACIDS RESEARCH, vol. 11, no. 12, June 25, 1983, Washington D.C.<br>A. SINGH et al. "Saccharomyces cerevisiae contains two discrete genes coding for the α-factor pheromone"<br>pages 4049-4063<br>* Page 4049, abstract *<br>---- | 4 | TECHNICAL FIELDS SEARCHED (Int. Cl 4)<br>C 12 N |

The present search report has been drawn up for all claims

| Place of search | Date of completion of the search | Examiner |
|---|---|---|
| VIENNA | 03-12-1985 | WOLF |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

EPO Form 1503 03 82